(19) Europäisches Patentamt
European Patent Office
Office européen des brevets

(11) EP 1 482 018 A1

(12) EUROPÄISCHE PATENTANMELDUNG

(43) Veröffentlichungstag:
**01.12.2004 Patentblatt 2004/49**

(21) Anmeldenummer: **04010356.6**

(22) Anmeldetag: **30.04.2004**

(51) Int Cl.[7]: **C09K 19/34**, C07D 319/06, C07C 47/47, C07C 47/277, C07C 47/575

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **27.05.2003 DE 10324313**

(71) Anmelder: **MERCK PATENT GmbH**
**64293 Darmstadt (DE)**

(72) Erfinder:
- **Poetsch, Eike Dr.**
  **64367 Mühltal (DE)**
- **Binder, Werner**
  **64807 Dieburg (DE)**
- **Meyer, Volker**
  **64846 Gross-Zimmern (DE)**
- **Gürtler, Stephan Dr.**
  **64347 Griesheim (DE)**
- **Eckstein, Jürgen**
  **64380 Rossdorf (DE)**
- **Schwarz, Michael Dr.**
  **64331 Weiterstadt (DE)**

(54) **Aldehyde mit Difluoroxymethylen-Brücke**

(57) Die Erfindung betrifft Aldehyde mit Difluoroxymethylen-Brücke der allgemeinen Formel I

$$OHC\text{-}(A^{11})_m\text{-}Z^{11}\text{-}(A^{12}\text{-}Z^{12})_n\text{-}(A^{13}\text{-}Z^{13})_p\text{-}A^{14}$$

und ihre Verwendung zur Herstellung von 1,3-Dioxanverbindungen.

EP 1 482 018 A1

## Beschreibung

Die Erfindung betrifft Aldehyde mit Difluoroxymethylen-Brücke und ihre Verwendung zur Herstellung von 1,3-Dioxanverbindungen.

Mesogene Verbindungen mit Difluoroxymethylen-Brücke ($CF_2O$-Brücke) spielen als Bestandteile flüssigkristalliner Mischungen zur Verwendung beispielsweise in elektrooptischen Anzeigevorrichtungen eine bedeutende Rolle (siehe zum Beispiel EP 844 229 A1 und EP 881 221 A1). Ihre Synthese gestaltet sich jedoch oft schwierig, und für einzelne Verbindungen müssen die Reaktionsparameter der Herstellungsverfahren zumeist gezielt und aufwendig angepaßt werden. Ferner nutzen die bekannten Herstellungsverfahren lineare Syntheserouten, bei denen die $CF_2O$-Gruppe erst auf einer der letzten Stufen einer oft vielstufigen Synthese in dem ansonsten bereits komplett aufgebauten mesogenen Molekül eingeführt werden kann, zum Beispiel durch Umwandlung einer Ester-Funktion in zwei Schritten (siehe EP 844 229 A1). Diese Synthesewege erweisen sich oft als unflexibel und nur in engen Grenzen auf die Synthese anderer Moleküle mit Difluoroxymethylen-Gruppe übertragbar. Dies trifft insbesondere auf solche Moleküle mit $CF_2O$-Brücke zu, die z.B. gegenüber Lewis- oder Protonensäuren empfindliche Strukturelemente - etwa cyclische oder acyclische Acetalstrukturen - aufweisen.

Hingegen sind erfolgreiche Strategien zur konvergenten Synthese komplexer mesogener Verbindungen mit $-CF_2O$-Gruppe aus vergleichsweise einfach zugänglichen und vielseitig einsetzbaren Vorstufen (Synthonen) bislang nicht bekannt, zumal es in der Literatur an solchen Vorstufen fehlt.

Eine Aufgabe der vorliegenden Erfindung besteht daher darin, Vorstufen zur Synthese von Verbindungen mit Difluoroxymethylen-Brücke bereitzustellen.

Diese Aufgabe wird gelöst durch Bereitstellung von Aldehyden gemäß der allgemeinen Formel I

$$OHC-(A^{11})_m-Z^{11}-(A^{12}-Z^{12})_n-(A^{13}-Z^{13})_p-A^{14} \qquad I$$

wobei

$A^{11}$, $A^{12}$ und $A^{13}$ unabhängig voneinander für

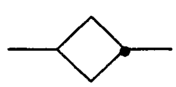

,

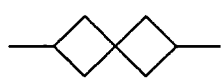

,

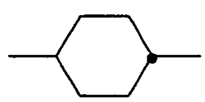

oder

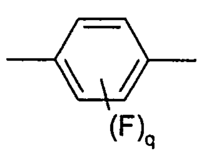

stehen;

$A^{14}$ für

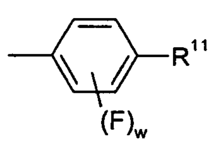

steht;

| | |
|---|---|
| $Z^{11}$, $Z^{12}$ und $Z^{13}$ | unabhängig voneinander eine Einfachbindung, $-CH_2CH_2-$, $-CF_2CH_2-$, $-CH_2CF_2-$, $-CF_2CF_2-$, - CH=CH-, -C≡C-, -CO-O-, -O-CO-, $-CH_2O-$, $-OCH_2-$ oder $-CF_2O$ bedeuten, wobei wenigstens eines von $Z^{11}$, $Z^{12}$ und $Z^{13}$ $-CF_2O-$ bedeutet; |
| m, n und p | unabhängig voneinander 0 oder 1 bedeuten; |
| q und w | unabhängig voneinander 0, 1, 2, 3 oder 4 bedeuten; |
| $R^{11}$ | H, F, Cl, Br, I, CN, -NCS, einen O-Aralkylrest oder einen unsubstituierten oder mit F, Cl, Br, I und/oder -CN ein- oder mehrfach substituierten Alkylrest mit 1 bis 15 Kohlenstoffatomen bedeutet, wobei in diesem Rest eine oder mehrere $CH_2$-Gruppen jeweils unabhängig voneinander durch -C≡C-, -CH=CH-, -O-, -S-, -CO-, -CO-O- oder -O-CO- so ersetzt sein können, dass Heteroatome nicht direkt miteinander verknüpft sind. |

Die erfindungsgemäßen Aldehyde zeichnen sich durch ihre vielseitige Verwendbarkeit zur Synthese komplexerer Verbindungen mit Difluoroxymethylen-Brücke aus. Ferner sind sie gut und in hoher Ausbeute aus vergleichsweise einfachen Ausgangsverbindungen zugänglich.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen; dieser Rest ist unsubstituiert oder einfach oder mehrfach gleich oder verschieden mit Fluor, Chlor, Brom, Iod und/oder Cyano substituiert.

Sofern es sich bei diesem Alkylrest um einen gesättigten Rest handelt, wird er auch als "Alkanyl" bezeichnet. Ferner umfaßt der Ausdruck "Alkyl" auch unsubstituierte oder entsprechend mit F, Cl, Br, I und/oder -CN ein- oder mehrfach gleich oder verschieden substituierte Kohlenwasserstoffreste, in denen eine oder mehrere $CH_2$-Gruppen derart durch -O- ("Alkoxy", "Oxaalkyl"), -S- ("Thioalkyl"), -CH=CH- ("Alkenyl"), -C≡C- ("Alkinyl"), -CO-Ooder -O-CO-ersetzt sein können, dass Heteroatome (O, S) nicht direkt miteinander verknüpft sind. Vorzugsweise ist Alkyl ein geradkettiger oder verzweigter, unsubstituierter oder substituierter Alkanyl-, Alkenyl- oder Alkoxyrest mit 1, 2, 3, 4, 5, 6, 7 oder 8 Kohlenstoffatomen. Sofern Alkyl einen Alkanylrest bedeutet, ist dieser bevorzugt Methyl, Ethyl, n-Propyl, i-Propyl, n-Butyl, i-Butyl, t-Butyl, n-Pentyl, Neopentyl, n-Hexyl, n-Heptyl, n-Octyl; $CF_3$, $CHF_2$, $CH_2F$; $CF_2CF_3$. Besonders bevorzugt ist der Alkanylrest geradkettig und unsubstituiert oder mit F substituiert.

Da in einem Alkylrest gemäß dieser Erfindung eine oder mehrere $CH_2$-Gruppen durch -O- ersetzt sein können, umfaßt der Ausdruck "Alkyl" auch "Alkoxy"- beziehungsweise "Oxaalkyl"-Reste. Unter Alkoxy ist ein O-Alkyl-Rest zu verstehen, in dem das Sauerstoffatom direkt mit der durch den Alkoxyrest substituierten Gruppe oder dem substituierten Ring verbunden ist und Alkyl wie oben definiert ist; vorzugsweise ist Alkyl dann Alkanyl oder Alkenyl. Bevorzugte Alkoxyreste sind Methoxy, Ethoxy, Propoxy, Butoxy, Pentoxy, Hexoxy, Heptoxy und Octoxy, wobei jeder dieser Reste auch substituiert sein kann, und zwar vorzugsweise mit einem oder mehreren Fluoratomen. Besonders bevorzugt ist Alkoxy $-OCH_3$, $-OC_2H_5$, $-O-n-C_3H_7$, $-O-n-C_4H_9$, $-O-t-C_4H_9$, $-OCF_3$, $-OCHF_2$, $-OCH_2F$ or $-OCHFCHF_2$. Im Zusammenhang mit der vorliegenden Erfindung bedeutet der Ausdruck "Oxaalkyl" Alkylreste, in denen wenigstens eine nicht-terminale $CH_2$-Gruppe durch -O- derart ersetzt ist, dass keine benachbarten Heteroatome (O, S) vorliegen. Vorzugsweise umfaßt Oxaalkyl geradkettige Reste der Formel $C_aH_{2a+1}-O-(CH_2)_b-$, wobei a und b jeweils unabhängig voneinander 1, 2, 3, 4, 5, 6, 7, 8, 9 oder 10 bedeuten; besonders bevorzugt ist a eine ganze Zahl von 1 bis 6 und b 1 oder 2.

Sofern in einem wie oben definierten Alkylrest eine oder mehrere $CH_2$-Gruppen durch Schwefel ersetzt sind, liegt ein "Thioalkyl"-Rest vor. Vorzugsweise umfaßt "Thioalkyl" einen geradkettigen Rest der Formel - $C_aH_{2a+1}-S-(CH_2)_b-$, wobei a 1, 2, 3, 4, 5, 6, 7, 8, 9 oder 10 ist und b 0, 1, 2, 3, 4, 5, 6, 7, 8, 9 oder 10 ist; besonders bevorzugt ist a eine ganze Zahl von 1 bis 6 und b 0, 1 oder 2. Der Thioalkylrest kann ebenfalls mit F, Cl, Br, I und/oder -CN substituiert sein und ist vorzugsweise unsubstituiert.

Im Zusammenhang der vorliegenden Erfindung bedeutet der Ausdruck "Alkenyl" einen wie oben definierten Alkylrest, in dem eine oder mehrere - CH=CH-Gruppen vorhanden sind. Sofern zwei -CH=CH-Gruppen in dem Rest vorhanden sind, kann dieser auch als "Alkadienyl" bezeichnet werden. Ein Alkenylrest kann 2 bis 15 (d.h. 2, 3, 4, 5, 6, 7, 8, 9, 10, 11, 12, 13, 14 oder 15) Kohlenstoffatome enthalten und ist verzweigtkettig oder vorzugsweise geradkettig. Der Rest ist unsubstituiert oder ein- oder mehrfach gleich oder verschieden mit F, Cl, Br, I und/oder CN substituiert. Ferner können eine oder mehrere $CH_2$-Gruppen jeweils unabhängig voneinander durch -O-, -S-, -C≡C-, -CO-O-, -OC-O- so ersetzt sein, dass Heteroatome (O, S) nicht direkt miteinander verbunden sind. Falls die CH=CH-Gruppe an beiden Kohlenstoffatomen einen anderen Rest als Wasserstoff trägt, etwa wenn sie eine nicht-terminale Gruppe ist, kann die CH=CH-Gruppe in zwei Konfigurationen vorliegen, nämlich als E-Isomer und als Z-Isomer. Im allgemeinen ist das E-Isomer (trans) bevorzugt. Vorzugsweise enthält der Alkenylrest 2, 3, 4, 5, 6 oder 7 Kohlenstoffatome und

bedeutet Vinyl, 1E-Propenyl, 1 E-Butenyl, 1E-Pentenyl, 1E-Hexenyl, 1 E-Heptenyl, 2-Propenyl, 2E-Butenyl, 2E-Pentenyl, 2E-Hexenyl, 2E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl. Besonders bevorzugte Alkenylreste sind Vinyl, 1E-Propenyl und 3E-Butenyl.

Falls in einem Alkylrest eine oder mehrere $CH_2$-Gruppen durch -C≡Cersetzt sind, liegt ein Alkinylrest vor. Auch die Ersetzung von einer oder mehreren $CH_2$-Gruppen durch -CO-O- or -O-CO- ist möglich. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)-ethyl, 3-(Methoxycarbonyl)-propyl, 3-(Ethoxy-carbonyl)-propyl oder 4-(Methoxycarbonyl)-butyl.

Im Zusammenhang der vorliegenden Erfindung steht der Ausdruck "O-Aralkyl" für einen Aryl-Alkyl-O-Rest, d.h. für einen Rest, in dem ein Aryl-Substituent über eine Alkyl-Sauerstoff-Brücke mit einem Atom, einer Kette, einem anderen Rest oder einer funktionellen Gruppe verknüpft ist. Unter einem Aryl-Substituenten versteht man dabei einen aromatischen Kohlenwasserstoff mit 6 bis 18 Kohlenstoffatomen, der gegebenenfalls mit Halogen, Amino-, Nitro-, Alkanyl- und/oder Alkoxy-Resten substituiert ist, insbesondere einen Phenyl- und einen Naphthylrest. Die Alkyl-Sauerstoff-Brücke ist vorzugsweise gesättigt. Insbesondere handelt es sich um Methylen-O ($-CH_2-O-$) und Ethylen-O ($-CH_2CH_2-O-$). Bevorzugte Beispiele eines O-Aralkylrests sind Benzyl-O- und Phenethyl-O-.

Sofern Reste oder Substituenten der erfindungsgemäßen Aldehyde beziehungsweise die erfindungsgemäßen Aldehyde selbst als optisch aktive oder stereoisomere Reste, Substituenten beziehungsweise Verbindungen vorliegen können, weil sie beispielsweise ein asymmetrisches Zentrum aufweisen, so sind diese von der vorliegenden Erfindung mit umfaßt. Dabei ist es selbstverständlich, dass die erfindungsgemäßen Aldehyde der allgemeinen Formel I in isomerenreiner Form, zum Beispiel als reine Enantiomeren, Diastereomeren, E- beziehungsweise Z-Isomeren, trans- beziehungsweise cis-Isomeren, oder als Gemisch mehrerer Isomeren in jedem beliebigen Verhältnis, zum Beispiel als Racemat, E/Z-Isomerengemisch oder cis/trans-Isomerengemisch, vorliegen können.

Bei den erfindungsgemäßen Aldehyden handelt es sich um Verbindungen, die im allgemeinen bei Raumtemperatur über lange Zeit stabil sind und in flüssiger oder fester Form vorliegen.

Die erfindungsgemäßen Aldehyde zeichnen sich dadurch aus, dass sie wenigstens eine Difluoroxymethylen-Brücke besitzen und an dem der Aldehyd-Funktion entgegengesetzten Ende des Moleküls einen substituierten Phenylrest aufweisen. Dabei ist dieser endständige Rest $A^{14}$ ein substituierter Phenylrest der Formel

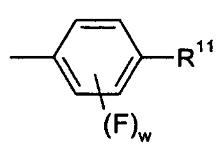

und bevorzugt ein substituierter Phenylrest der Formel

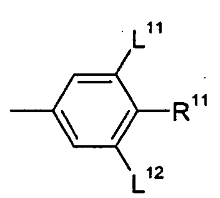

,

wobei w 0, 1, 2, 3 oder 4 ist, $L^{11}$ und $L^{12}$ unabhängig voneinander H oder F bedeuten und $R^{11}$ H, F, Cl, Br, I, CN, -NCS, einen O-Aralkylrest oder einen unsubstituierten oder mit F, Cl, Br, I und/oder -CN ein- oder mehrfach substituierten Alkylrest mit 1 bis 15 Kohlenstoffatomen bedeutet, wobei in diesem Rest eine oder mehrere $CH_2$-Gruppen jeweils unabhängig voneinander durch -C≡C-, -CH=CH-, -O-, -S-, -CO-, -CO-O- oder -O-CO- so ersetzt sein können, dass Heteroatome nicht direkt miteinander verknüpft sind. Bevorzugt steht $R^{11}$ dabei für H oder einen polaren Rest, insbesondere für F, Cl, $OCF_3$, $OCHF_2$ oder $CF_3$. Es ist ferner besonders bevorzugt, dass entweder $L^{11}$ oder $L^{12}$ oder beide Substituenten, $L^{11}$ und $L^{12}$, F bedeuten.

In bestimmten bevorzugten Ausführungsformen der erfindungsgemäßen Aldehyde bedeuten $Z^{11}$, $Z^{12}$ und $Z^{13}$ in der obigen Formel I jeweils unabhängig voneinander eine Einfachbindung, $-CH_2CH_2-$, $-CF_2CH_2-$, $-CH_2CF_2-$,

$-CF_2CF_2-$ oder $-CF_2O-$, wobei wenigstens eines von $Z^{11}$, $Z^{12}$ und $Z^{13}$ $-CF_2O-$ bedeutet. Wenn dabei zum Beispiel p und n in der obigen Formel I beide null sind und somit der erfindungsgemäße Aldehyd der Formel I keine $A^{12}$-$Z^{12}$-Gruppe und keine $A^{13}$-$Z^{13}$-Gruppe aufweist, ist $Z^{11}$ $-CF_2O-$. Wenn beispielsweise n 1 ist und p 0 ist, dann ist entweder $Z^{11}$ oder $Z^{12}$ eine Difluoroxymethylen-Brücke und die jeweils andere Brücke ($Z^{12}$ beziehungsweise $Z^{11}$) bedeutet dann bevorzugt eine Einfachbindung, $-CH_2CH_2-$, $-CF_2CH_2-$, $-CH_2CF_2-$ oder $-CF_2CF_2-$, oder beide, $Z^{11}$ und $Z^{12}$, stehen für jeweils eine Difluoroxymethylen-Brücke. Besonders bevorzugt stehen $Z^{11}$, $Z^{12}$ und $Z^{13}$ unabhängig voneinander für eine Einfachbindung oder $-CF_2O-$, wobei wenigstens eine der Brücken $Z^{11}$, $Z^{12}$ und $Z^{13}$ $-CF_2O$bedeutet.

In einer weiteren bevorzugten Ausführungsform der vorliegenden Erfindung ist $Z^{11}$ in Formel I $-CF_2O-$. Sofern m in Formel I null ist - was für noch eine weitere bevorzugte Ausführungsform der Erfindung gilt -, so ist in dieser Ausführungsform zwischen der funktionellen CHO-Gruppe und der Brücke $Z^{11}$ kein Ring $A^{11}$ vorhanden und die $-CF_2O$-Gruppe direkt mit der Carbonyl-Funktion des erfindungsgemäßen Aldehyds der Formel I verbunden.

Es ist ferner besonders bevorzugt, dass n und p beide 0 sind, so dass $Z^{11}$ eine Difluoroxymethylen-Brücke darstellt.

Besonders bevorzugte Aldehyde der vorliegenden Erfindung sind aus der Gruppe ausgewählt, die aus folgenden Aldehyden besteht, welche einen Ring $A^{14}$=

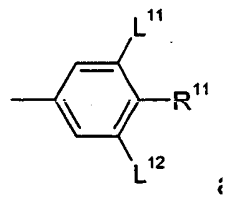

aufweisen:

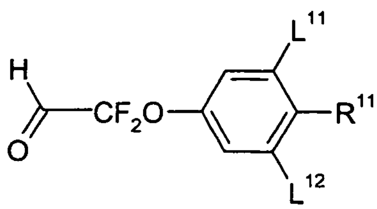

I-A

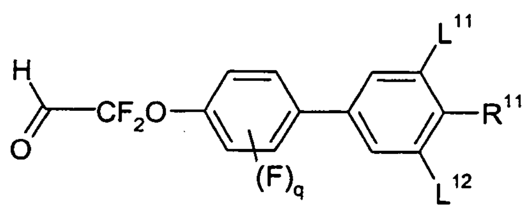

I-B

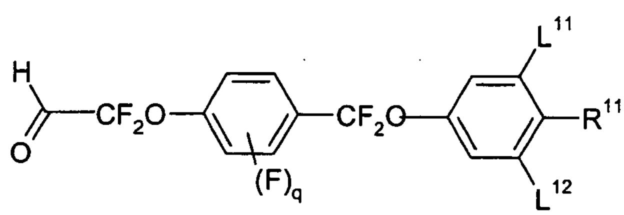

I-C

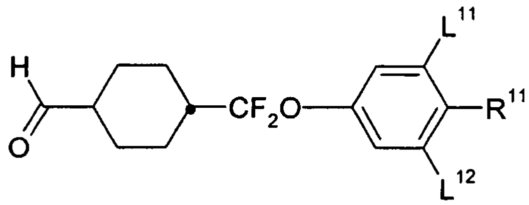

I-D

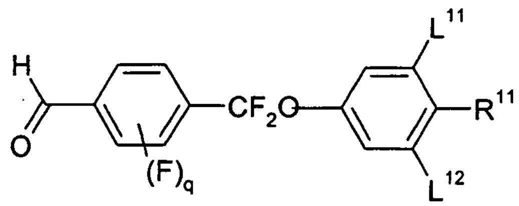

I-E

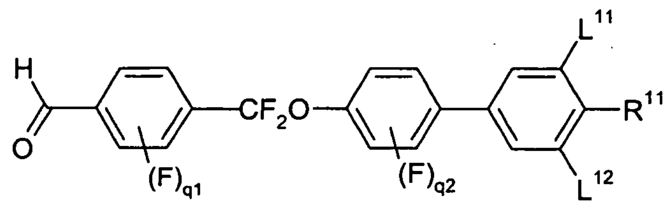

I-F

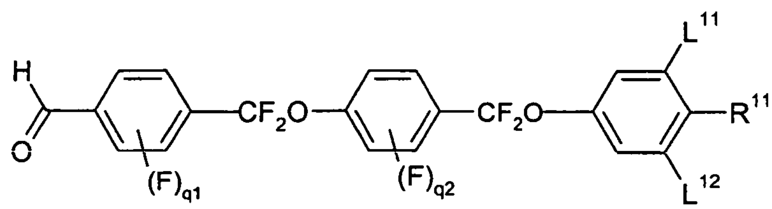

I-G

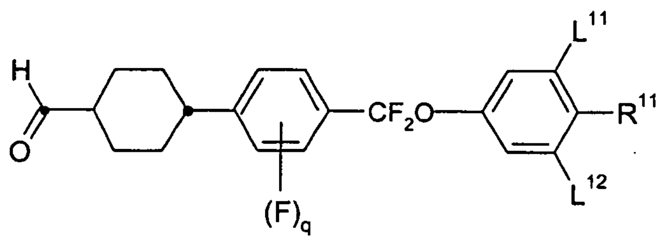

I-H

In den oben angegebenen Formeln I-A bis I-H hat der Rest $R^{11}$ die oben für Formel I angegebene Bedeutung, während $L^{11}$ und $L^{12}$ unabhängig voneinander für H oder F stehen. Die Indizes q, q1 und q2 sind unabhängig voneinander 0, 1, 2, 3 oder 4, d.h. die entsprechenden 1,4-Phenylenringe können jeweils unabhängig voneinander kein Fluoratom oder in jeder freien Ringposition (2-, 3-, 5- und 6-Stellung) je 1 Fluoratom und insgesamt bis zu 4 Fluoratome aufweisen; bevorzugt stehen q, q1 und q2 unabhängig voneinander für 0, 1 oder 2 und sind die Fluor-Substituenten - sofern sie im Molekül vorhanden sind - in 3- beziehungsweise 5-Position des 1,4-Phenylenrings (d.h. sie weisen dieselbe Orientierung auf wie die Substituenten $L^{11}$ beziehungsweise $L^{12}$).

Die Aldehyde der Formeln I-A, I-B und I-C entsprechen der allgemeinen Formel I in der Weise, dass m null ist und $Z^{11}$ $-CF_2O-$ bedeutet. In Formel I-A sind auch n und p null. In Formel I-B ist p null, während n 1 ist, $A^{12}$ für einen gegebenenfalls mit Fluor substituierten 1,4-Phenylenring steht und $Z^{12}$ eine Einfachbindung darstellt; Formel I-C unterscheidet sich von Formel I-B darin, dass $Z^{12}$ keine Einfachbindung, sondern eine Difluoroxymethylen-Brücke darstellt.

Die Aldehyde der Formeln I-D und I-E leiten sich von der allgemeinen Formel I dadurch ab, dass m 1 ist, n und p beide 0 sind und $Z^{11}$ die Difluoroxymethylen-Brücke bildet; $A^{11}$ ist entweder ein (bevorzugt trans-substituierter) 1,4-Cyclohexylenring (I-D) oder ein optional substituierter 1,4-Phenylenring (I-E).

Bei den Aldehyden der Formeln I-F und I-G sind m und n jeweils 1, während p null ist; sowohl $A^{11}$ als auch $A^{12}$ stellen jeweils einen 1,4-Phenylenring dar, während $Z^{12}$ eine Einfachbindung (I-F) beziehungsweise eine $CF_2O$-Brücke (I-G) darstellt.

Die Aldehyde der Formel I-H entsprechen der allgemeinen Formel I in der Weise, dass m und n 1 sind und p 0 ist, $Z^{11}$ eine Einfachbindung und $Z^{12}$ die $CF_2O$-Brücke darstellen, $A^{11}$ ein (bevorzugt trans-substituierter) 1,4-Cyclohexylenring ist und $A^{12}$ ein gegebenenfalls fluorierter 1,4-Phenylenring ist.

In den Aldehyden der Formeln I-A bis I-H ist $R^{11}$ bevorzugt ein polarer Rest, besonders bevorzugt F, Cl, $OCF_3$, $OCHF_2$ oder $CF_3$, insbesondere F. $L^{11}$ und $L^{12}$ sind unabhängig voneinander H oder F, bevorzugt ist wenigstens eines von $L^{11}$ und $L^{12}$ F und besonders bevorzugt sind beide Substituenten $L^{11}$ und $L^{12}$ F. Die Indizes q, q1 und q2 sind bevorzugt jeweils 2.

Unter den bevorzugten Aldehyden der Formeln I-A bis I-H sind besonders bevorzugt Aldehyde der Formeln I-A, I-D, I-E und I-H, insbesondere Aldehyde der Formel I-D.

Beispiele besonders bevorzugter Aldehyde der Formel I-A sind:

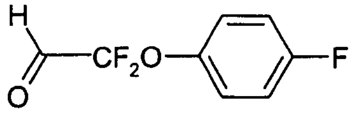

I-A1

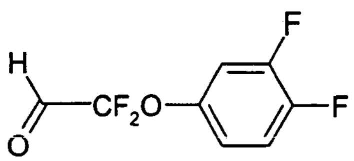

I-A2

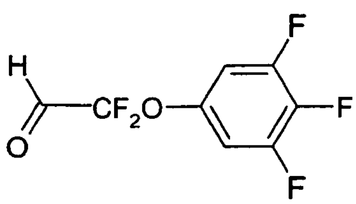

I-A3

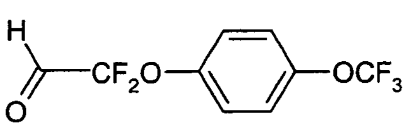

I-A4

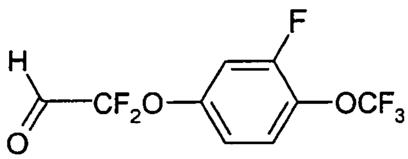

I-A5

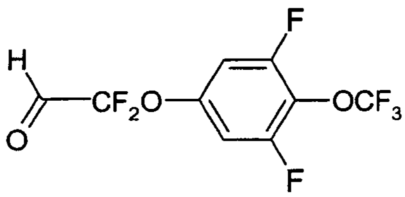

I-A6

Beispiele besonders bevorzugter Aldehyde der Formel I-D sind:

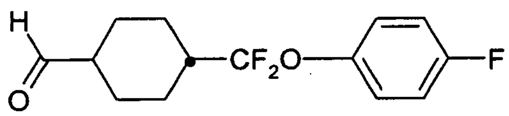

I-D1

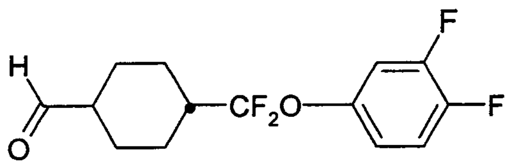

I-D2

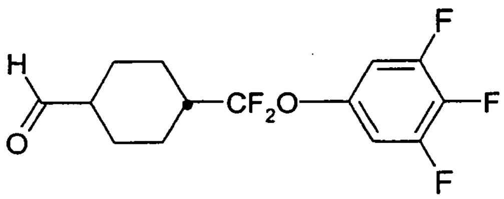

I-D3

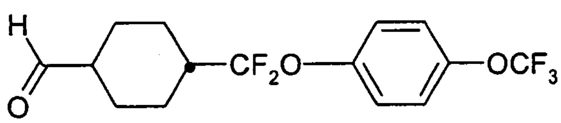

I-D4

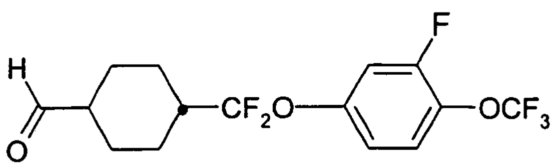

I-D5

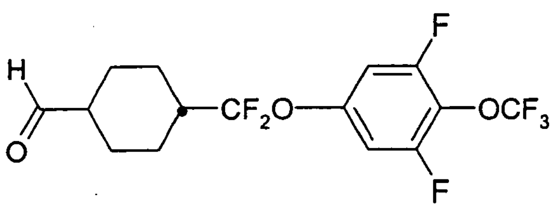

I-D6

Beispiele besonders bevorzugter Aldehyde der Formel I-E sind:

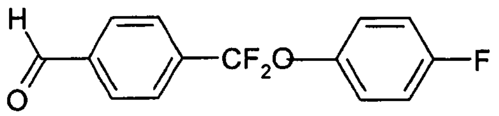

I-E1

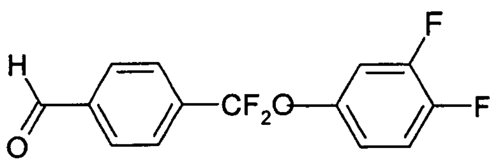

I-E2

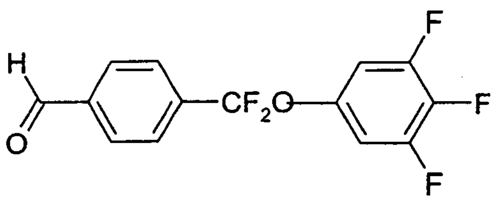

I-E3

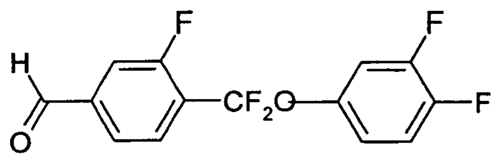

I-E4

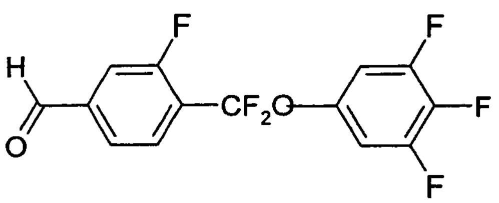

I-E5

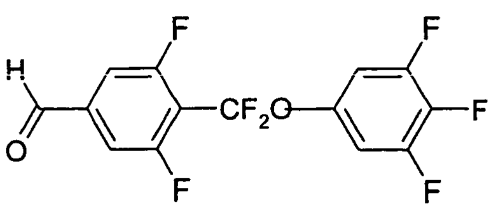

I-E6

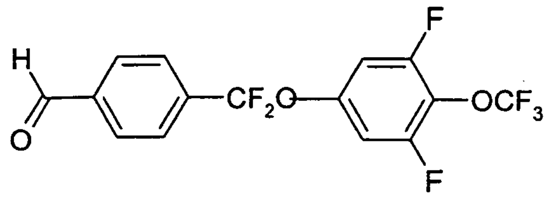

I-E7

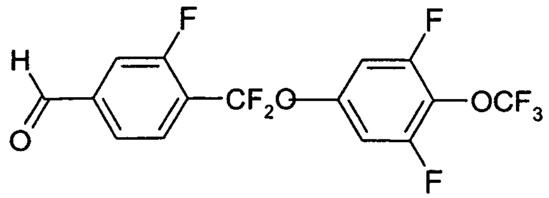

I-E8

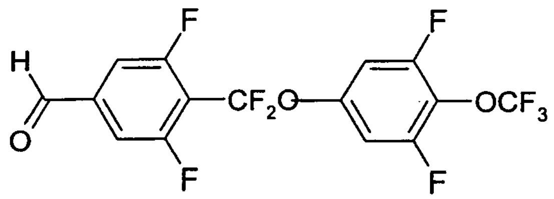

I-E9

Beispiele besonders bevorzugter Aldehyde der Formel I-H sind:

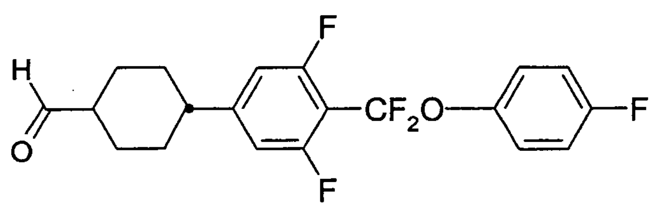

I-H1

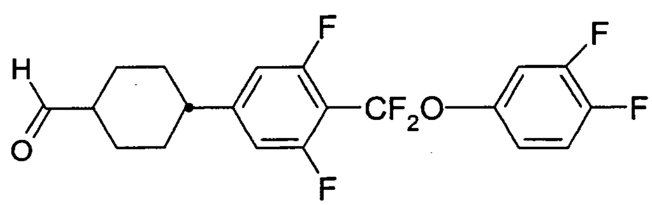

I-H2

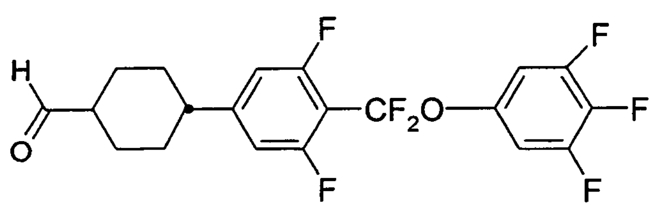

I-H3

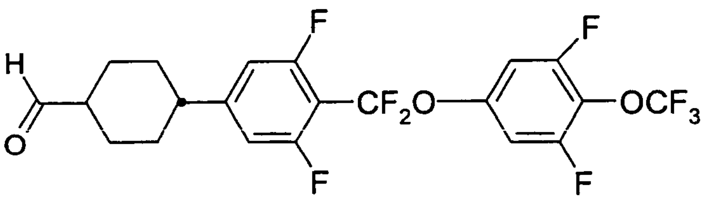

I-H4

Die erfindungsgemäßen Aldehyde werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

So sind Aldehyde der Formel I-A gemäß dem folgenden Schema 1 in einfacher Weise und guten Ausbeuten aus käuflich erhältlichen beziehungsweise literaturbekannten Ausgangsverbindungen zugänglich:

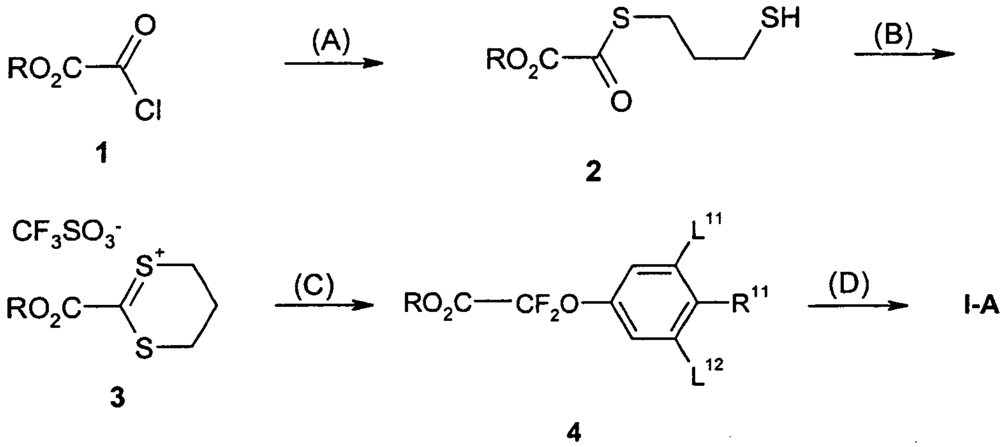


1 2 3 4 I-A

**Schema 1**

Dabei erfolgt die Umsetzung von **1** (R ist ein Alkyl- oder Benzylrest) zu **2** in Schritt (A) mit NaS-$(CH_2)_3$-SH. **2** wird mit Trifluormethansulfonsäure (in Analogie zu den Verfähren von P. Kirsch et al., Angew. Chem. **2001,** *113*, 1528; siehe auch WO 01/64667) in Schritt (B) zu **3** umgesetzt. In Schritt (C) erfolgt dann die oxidative Fluordesulfurierung (gemäß P. Kirsch et al., Angew. Chem. **2001,** *113*,1528, und WO 01/64667), indem **3** zunächst bei tiefen Temperaturen mit $NEt_3 \cdot 3$ HF (Et = Ethyl) und einem Phenol der Formel

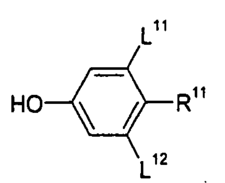

,

dann mit 1,3-Dibrom-5,5-dimethylhydanthoin (DBH) oder N-Bromsuccinimid (NBS) oder Brom und schließlich mit wäßriger Lauge zu **4** umgesetzt wird. In Schritt (D) erfolgt die Einführung der Aldehyd-Funktion unter Ergeben der erfindungsgemäßen Verbindung I-A, entweder durch direkte Reduktion des Esters mit einem geeigneten Reduktionsmittel wie DIBAL-H (Diisobutylaluminiumhydrid) in einem inerten Solvens, zum Beispiel n-Heptan, oder über die Reduktion des Esters zum entsprechenden Alkohol und anschließende Oxidation zum Aldehyd mit einem geeigneten Oxidationsmittel, zum Beispiel mit Dess-Martin-Reagenz.

Eine alternative Synthese ist in Schema 2 wiedergegeben:

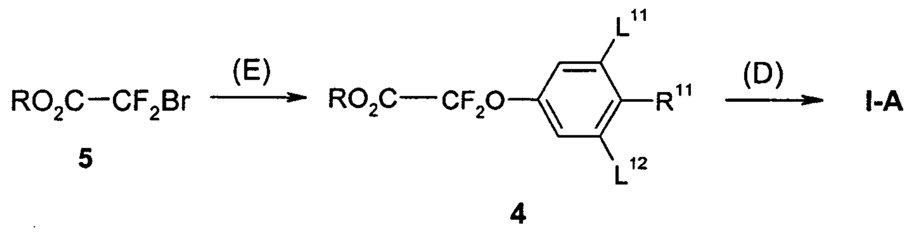

**Schema 2**

Verbindung **5** (mit R = unsubstituiertes Alkanyl oder Benzyl) wird mit einem Phenolat der Formel

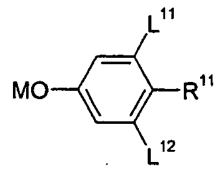

(mit M = Na, K oder Cs) zu der aus Schema 1 bekannten Verbindung **4** umgesetzt, die dann gemäß Schema 1 in den erfindungsgemäßen Aldehyd der Formel I-A überführt wird. Die Durchführung des Verfahrensschritts (E) kann dabei auf unterschiedliche Art und Weise erfolgen. Eine Variante besteht darin, das Phenolat mit **4** in einem etherischen Lösungsmittel, zum Beispiel Tetrahydrofuran (THF), in Gegenwart von Hexamethylphosphorsäuretriamid (HMPT) umzusetzen. Bei einer anderen Variante erfolgt die Umsetzung von Phenolat mit **4** unter Katalyse mit einem $Pd^0$-Katalysator, der beispielsweise in situ aus Palladiumdichlorid-Bis(tricyclohexylphosphan) gebildet werden kann.

In Analogie zu den Schemata 1 und 2 sind auch die erfindungsgemäßen Aldehyde der Formel I-B zugänglich, indem in Verfahrensschritt (C) in Schema 1 das Biphenyl der folgenden Formel

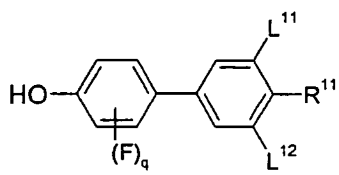

(worin $R^{11}$, $L^{11}$, $L^{12}$ und q die für Formel I angegebenen Bedeutungen aufweisen) mit Verbindung **3** beziehungsweise in Verfahrensschritt (E) in Schema 2 das entsprechende Alkoholat mit Verbindung **5** umgesetzt wird.

Erfindungsgemäße Aldehyde der Formel I-C sind beispielsweise gemäß Schema 3 zugänglich:

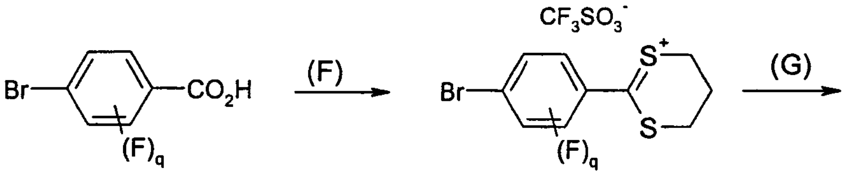


6 7

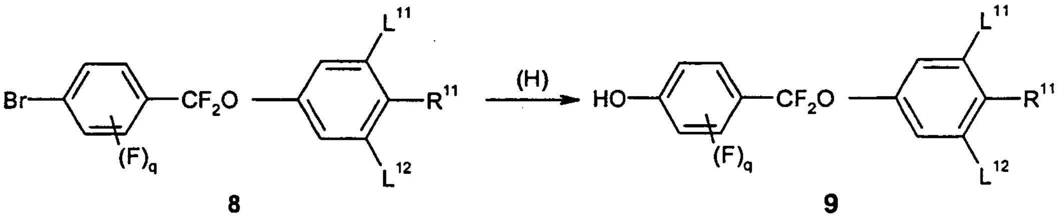


8 9

3 (aus Schema 1) + 9 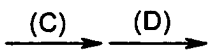 I-C


**Schema 3**

Die gegebenenfalls fluorierte 4-Brombenzoesäure (**6**) wird unter Bedingungen, die beispielsweise von P. Kirsch et al., Angew. Chem. **2001,** *113*, 1528, (siehe auch Erläuterungen zu Schema 1 oben) beschrieben worden sind, in Verbindung **7** überführt, welche ihrerseits einer oxidativen Fluordesulfurierung (gemäß P. Kirsch et al., Angew. Chem. **2001,** *113*, 1528, und WO 01/64667) ((G)) mit dem Phenol

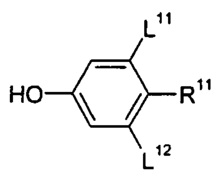

unter Bildung der Verbindung **8** mit Difluoroxymethylen-Brücke unterworfen wird. Die Umwandlung in das Phenol **9** erfolgt dann zum Beispiel durch Lithiierung mit n-Butyllithium, anschließende Bildung der entsprechenden Boronsäure mit Trimethylborat (oder einem anderen Boronsäurederivat) und abschließende Reaktion mit Wasserstoffperoxid unter Umwandlung der $-B(OH)_2$-Gruppe in die -OH-Gruppe des Phenols **9** (Schritt (H)). Dieses Phenol wird dann in einer zweiten oxidativen Fluordesulfurierungs-Reaktion unter vergleichbaren Bedingungen mit dem gemäß Schema 1 hergestellten Synthon **3** zu dem erfindungsgemäßen Aldehyd I-C umgesetzt.

Alternativ kann in Analogie zu dem Verfahren gemäß Schema 2 das Phenolat der Verbindung **9** mit Verbindung **5** in den Reaktionsschritten (E) und (D) in den Aldehyd I-C überführt werden.

Erfindungsgemäße Aldehyde der Formel I-D sind beispielsweise gemäß Schema 4 zugänglich:

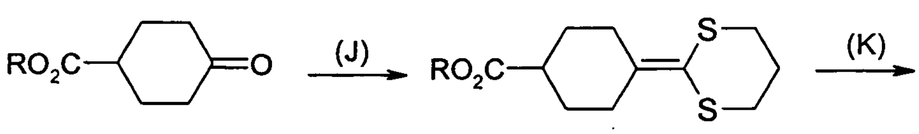


10 11

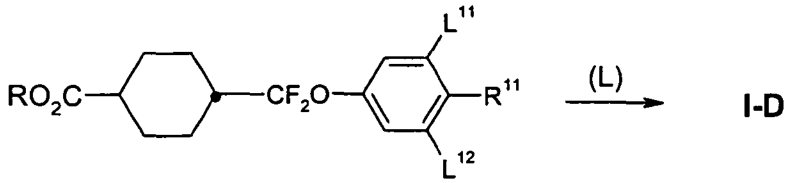

12

**Schema 4**

Die Umsetzung des 4-Cyclohexanoncarbonsäureesters **10** (R steht für einen unsubstituierten Alkanyl- oder Benzylrest) zu **11** ((J)) erfolgt gemäß dem oben erwähnten Verfahren von P. Kirsch et al., Angew. Chem. **2001**, *113*, 1528, oder auch in Anlehnung an das Verfahren von J. Mlynarski und A. Banaszek, Tetrahedron **55** (1999) 2785, durch Umsetzung mit Trimethylsilyl-1 ,3-dithian in THF in Gegenwart von n-Butyllithium in Hexan bei tiefen Temperaturen. Die anschließende oxidative Fluordesulfurierung von **11** zu **12** (vgl. auch P. Kirsch et al., Angew. Chem. **2001,** *113*, 1528) ((K)) und die abschließende Reduktion ((L)) zu dem erfindungsgemäßen Aldehyd I-D erfolgt in Analogie zu den oben dargelegten Syntheseverfahren. Sofern in einzelnen Fällen in Reaktionsschritt (K) neben dem bevorzugten trans-Cyclohexanderivat **12** auch das cis-Isomere erhalten wird, erfolgt die Umwandlung des cis-Isomeren in das trans-Isomere oder seine Entfernung aus dem Produktgemisch mittels bekannter Verfahren, zum Beispiel mittels Umkristallisation oder im Falle flüssiger Verbindungen mittels fraktionierter Destillation.

Die Isomerisierung kann auch durch Behandlung des cis/trans-Isomerengemischs mit einer Base, z.B. Natriumhydroxid, in einem geeignetem Lösungsmittel, z.B. einem Alkohol wie Methanol oder in Gemischen aus Methanol, Tetrahydrofuran und Wasser, durchgeführt werden.

Erfindungsgemäße Aldehyde der Formel I-E sind beispielsweise gemäß Schema 5 zugänglich:

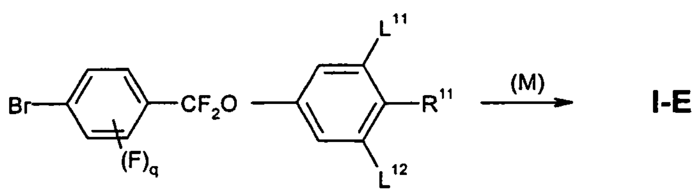

8

**Schema 5**

Das gemäß Schema 3 zugängliche Bromid **8** wird zum Beispiel durch einen Halogen-Metallaustausch mit einer metallorganischen Base, etwa n-Butyllithium, und Umsetzung mit einem Formylierungsreagenz wie N,N-Dimethylformamid oder N-Formylpiperidin in den erfindungsgemäßen Aldehyd I-E überführt. Eine bevorzugte Variante des Reaktionsschritts (M) besteht in dem Austausch des Broms in Verbindung **8** durch einen Halogen-Magnesiumaustausch mit Isopropylmagnesiumchlorid oder -bromid gegen MgCl beziehungsweise MgBr und Abfangen des so gebildeten Arylmagnesiumhalogenids mit dem Formylierungsreagenz. Alternativ kann das Bromid **8** mit CuCN/N-Methylpyrrolidon zunächst in das entsprechende Nitril überführt werden, das anschließend einer Reduktion mit einem geeigneten Reduktionsmittel wie DIBAL-H oder Bis(cyclopentadienyl)zirconiumchloridhydrid zum Aldehyd I-E unterworfen wird.

Erfindungsgemäße Aldehyde der Formel I-F sind beispielsweise gemäß Schema 6 zugänglich:

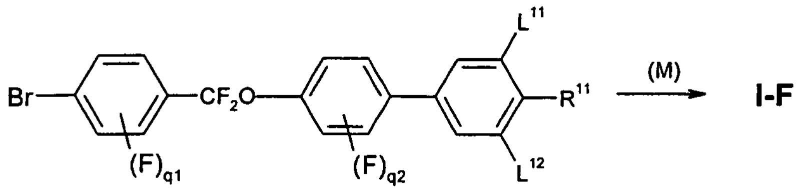


**8a**

## Schema 6

Dabei entspricht die Umsetzung des Bromids **8a** zu I-F jener des Bromids **8** zu I-E aus Schema 5; Bromid **8a** ist wie Bromid **8** gemäß Schema 3, Schritt (G), zugänglich, wobei jedoch Verbindung **7** mit der Biphenylverbindung

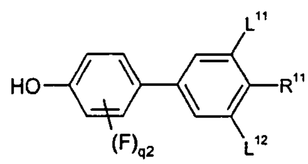

umgesetzt wird.

Erfindungsgemäße Aldehyde der Formel I-G sind beispielsweise in Analogie zur Herstellung der Aldehyde I-C durch entsprechende Umsetzung eines Bromids der Formel 7 mit einem Phenol der Formel 9 und anschließende Formylierung des Bromids über Brom-Metall-Austausch und Abfangreaktion mit einem Formylierungsreagenz zugänglich.

Erfindungsgemäße Aldehyde der Formel I-H sind zum Beispiel gemäß Schema 7 erhältlich:

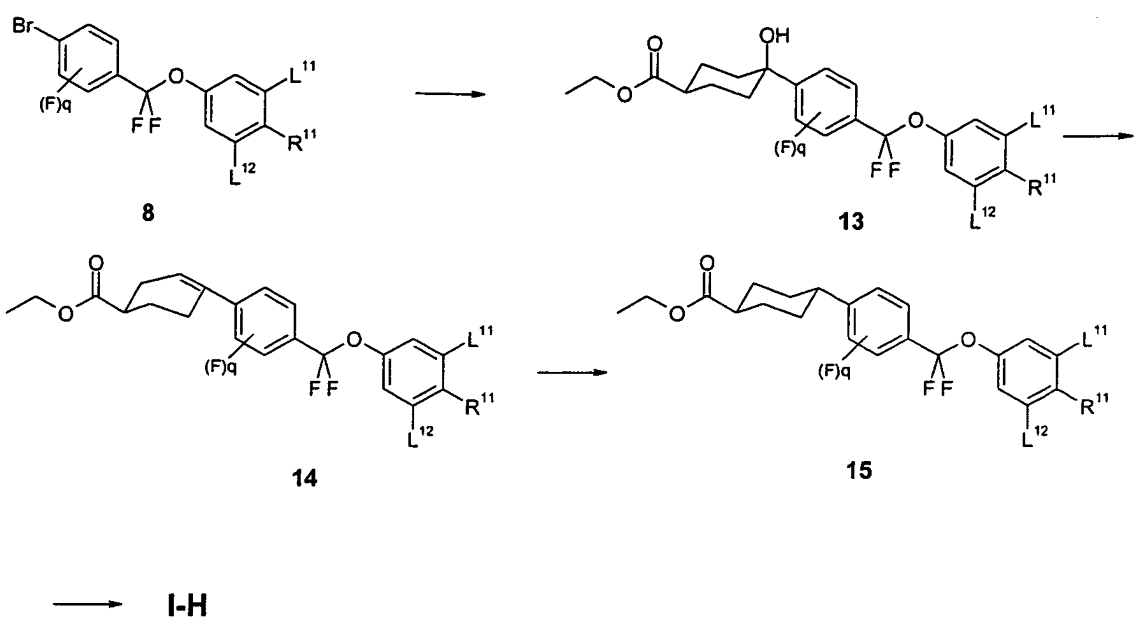


## Schema 7

Das Bromid 8 wird mit einer metalllorganischen Base, z.B. Isopropylmagnesiumchlorid, metalliert und mit Cyclohexan-4-oncarbonsäureethylester zum Ester **13** umgesetzt. Mit einem geeigneten wasserabspaltenden Reagenz, z.B. katalytischen Mengen einer starken Säure wie Schwefelsäure, erfolgt die Eliminierung zu **14**, das unter Übergangsmetall-Katalyse, z.B. mit Platin-Metall, zum Ester **15** hydriert wird. Falls erfoderlich kann die Isomerisierung von gegebenenfalls gebildetem cis-**15** zu trans-**15** nach üblichen Verfahren (z.B. Behandeln in einem geeigneten Lösungsmittel mit Base, z.B. NaOH) auf dieser Stufe erfolgen. Abschließend erfolgt die Reduktion mit beispielsweise Diisobutylaluminiumhydrid zu I-H. Auch auf der Endstufe kann eine gegebenenfalls erforderliche Isomerisierung von Produkt mit cis-Konfiguration am Cyclohexanring zu trans-I-H nach bekannten Verfahren (z.B. Behandeln mit methanolischer Natronlauge) erfolgen. Weitere erfindungsgemäße Aldehyde der Formel I sind - wie der Fachmann ohne weiteres erkennen kann - unter Anpassung der oben dargelegten und unter Anwendung weiterer im Stand der Technik bekannter Verfahren zugänglich. Mögliche beziehungsweise notwendige Abwandlungen können zum Beispiel Aldehyde der Formel I betreffen, in denen etwa $Z^{13}$ eine -CO-O-Gruppe darstellt. Dabei kann beispielsweise zunächst ein Aldehyd

$$OHC\text{-}A^{11}\text{-}CF_2O\text{-}A^{13}\text{-}Br$$

in Anlehnung an eines der oben ausführlicher dargelegten Verfahren aus dem Ester

$$Ethyl\text{-}O_2C\text{-}A^{11}\text{-}CF_2O\text{-}A^{13}\text{-}Br$$

aufgebaut werden, wobei das Bromid auf der "rechten" Molekülseite unter den Reaktionsbedingungen, unter denen der Ethylester auf der "linken" Molekülseite in die Aldehyd-Funktion überführt wird, inert ist. Nach Einführung der Aldehyd-Funktion und gegebenenfalls Schützen der Carbonylfunktion, etwa als Acetal, kann beispielsweise Halogen-Metall-Austausch, anschließende Umsetzung mit $CO_2$ und schließlich Veresterung mit einem Alkohol der Formel HO-$A^{14}$ nach Entfernen der gegebenenfalls vorhandenen Schutzgruppe zum erfindungsgemäßen Aldehyd

$$OHC\text{-}A^{11}\text{-}CF_2O\text{-}A^{13}\text{-}CO_2\text{-}A^{14}$$

erfolgen.

Die erfindungsgemäßen Aldehyde sind als Synthone für die Synthese komplexerer Verbindungen mit Difluoroxymethylen-Brücke, insbesondere mesogener Verbindungen, verwendbar. Ein weiterer Gegenstand der vorliegenden Erfindung besteht daher in einem Verfahren zur Herstellung von 1,3-Dioxanverbindungen unter Verwendung der erfindungsgemäßen Aldehyde der allgemeinen Formel I, die mit geeigneten 1,3-Diolen oder 1,3-bis-silylierten Derivaten dieser Diole umgesetzt werden.

Bei diesen Diolen beziehungsweise ihren bis-silylierten Derivaten handelt es sich vorzugsweise um solche der allgemeinen Formel II

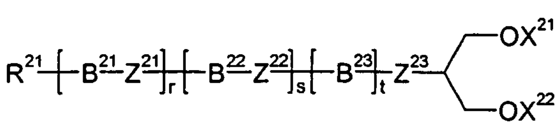

II

wobei

| | |
|---|---|
| r, s und t | unabhängig voneinander 0 oder 1 bedeuten; |
| $R^{21}$ | einen unsubstituierten oder mit F, Cl, Br, I und/oder - CN ein- oder mehrfach substituierten Alkylrest mit 1 bis 15 Kohlenstoffatomen bedeutet, wobei in diesem Rest eine oder mehrere $CH_2$-Gruppen jeweils unabhängig voneinander durch -C≡C-, - CH=CH-, -O-, -S-, -CO-, -CO-O- oder -O-CO- so ersetzt sein können, dass Heteroatome nicht direkt miteinander verknüpft sind; |
| $B^{21}$, $B^{22}$ und $B^{23}$ | unabhängig voneinander |

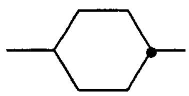

oder

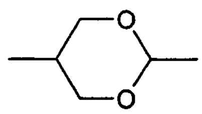

| | |
|---|---|
| | bedeuten; |
| $Z^{21}$, $Z^{22}$ und $Z^{23}$ | unabhängig voneinander eine Einfachbindung, $-CH_2CH_2-$, $-CF_2CH_2-$, $-CH_2CF_2-$, $-CF_2CF_2-$, - CH=CH- oder -C≡C- bedeuten; |
| $X^{21}$ und $X^{22}$ | H oder $SiR^{22}R^{23}R^{24}$ bedeuten, |
| wobei $R^{22}$, $R^{23}$ und $R^{24}$ | jeweils unabhängig voneinander ein Alkanylrest mit 1 bis 6 Kohlenstoffatomen ist und einer oder zwei der Reste $R^{22}$, $R^{23}$ und $R^{24}$ auch Phenyl sein können; |

so dass durch die Umsetzung mit den Aldehyden der Formel I Dioxanverbindungen der allgemeinen Formel III gebildet werden:

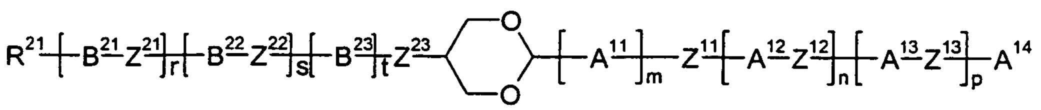 III

worin $R^{21}$, $A^{11}$, $A^{12}$, $A^{13}$, $A^{14}$, $B^{21}$, $B^{22}$, $B^{23}$, $Z^{11}$, $Z^{12}$, $Z^{13}$, $Z^{21}$, $Z^{22}$, $Z^{23}$, m, n, p, r, s und t die für die Formeln I und II angegebene Bedeutung besitzen.

In einer bevorzugten Ausführungsform des erfindungsgemäßen Verfahrens weist der eingesetzte Aldehyd die Struktur der Formel Ia auf:

$$OHC-(A^{11})_m-CF_2O-(A^{12}-Z^{12})_n-(A^{13}-Z^{13})_p-A^{14} \qquad \text{Ia}$$

wobei m entweder null ist oder 1 ist und $A^{11}$ zugleich einen 1,4-Cyclohexylenring darstellt; $A^{12}$, $A^{13}$, $A^{14}$, $Z^{12}$, $Z^{13}$, n und p weisen die in einem der Ansprüche 1 bis 7 angegebene Bedeutung auf.

Auch die 1,3-Diole und ihre bis-silylierten Derivate werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Herstellung zahlreicher 1,3-Diole und ihrer bis-silylierten Derivate ist unter anderem auch beschrieben in P. Kirsch und E. Poetsch, Adv. Mater. **1998,** *10*, 602, und dort zitierten Literaturstellen. Bevorzugte Dioxane der Formel III sind solche mit trans-Konfiguration des zentralen Dioxanrings

( 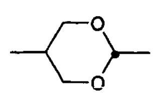 ).

Sie werden entweder unmittelbar in isomerenreiner Form bei der Umsetzung von Aldehyden der Formel I mit Verbindungen der Formel II oder nach Trennung der cis/trans-Isomeren, beispielsweise mittels Umkristallisation, erhalten.

Werden als Diole oder Diol-Derivate Verbindungen eingesetzt, die bereits einen trans-verknüpften Cyclohexanoder 1,3-Dioxan-Ring aufweisen, so bildet sich der gewünschte zentrale trans-1,3-Dioxan-Ring häufig in großem Isomerenüberschuß oder ausschließlich.

Die erfindungsgemäße Umsetzung der erfindungsgemäßen Aldehyde der Formel I mit Diolen oder Diol-Derivaten vorzugsweise der Formel II erfolgt unter für die Acetalbildung üblichen Bedingungen. Wird ein Diol der Formel II mit $X^{21} = X^{22} = H$ eingesetzt, so kann die Reaktion mit dem Aldehyd der Formel I in einem inerten Lösungsmittel üblicherweise bei Siedetemperatur unter Säurekatalyse und destillativer Entfernung des gebildeten Wassers erfolgen. Als Lösungsmittel eignen sich aromatische Lösungsmittel, insbesondere Toluol und Xylole. Als Säurekatalysator finden üblicherweise Lewis-Säuren sowie anorganische und organische Protonensäuren Verwendung, wobei p-Toluolsulfonsäure besonders bevorzugt ist. Die Reaktionszeit ist an sich nicht kritisch; sie beträgt üblicherweise zwischen 30 min und 18 h, in Abhängigkeit von der Geschwindigkeit der Kondensationsreaktion.

Bis-silylierte Diol-Derivate vorzugsweise der Formel II mit $X^{21} = X^{22} = SiR^{22}R^{23}R^{24}$ werden bevorzugt zur Umsetzung mit Aldehyden der Formel I eingesetzt, wenn die Verbindung der Formel I oder die Verbindung der Formel II oder beide säureempfindliche Gruppen enthalten, beispielsweise einen 1,3-Dioxan-Ring. Die Reaktion erfolgt dann vorzugsweise unter aprotischen Bedingungen in Gegenwart einer katalytischen Menge einer wasser- und protonenfreien Säure, zum Beispiel Trimethylsilyltriflat ($(Methyl)_3SiOSO_2CF_3$), in einem inerten Lösungsmittel, beispielsweise Dichlormethan, vorzugsweise bei Temperaturen unter Raumtemperatur, zum Beispiel bei -78 °C. Die Reaktionszeit ist auch hier an sich nicht kritisch; sie beträgt üblicherweise zwischen 15 oder 30 min und 18 h, in Abhängigkeit von der Geschwindigkeit der Umsetzung, die zumeist deutlich größer ist als bei den Diolen.

Bis-silylierte Verbindungen der Formel II sind aus den entsprechenden 1,3-Diolen nach üblichen Verfahren (siehe beispielsweise P. Kirsch und E. Poetsch, Adv. Mater. **1998,** *10*, 602, und dort zitierte Literaturstellen) zugänglich. Besonders bevorzugt sind jene Diol-Derivate der Formel II, die Trimethylsilyl-Schutzgruppen aufweisen ($X^{21} = X^{22}$ = Trimethylsilyl, $SiMe_3$). Ihre Einführung gelingt zum Beispiel durch Reaktion des korrespondierenden Diols mit überschüssigem Trimethylsilylchlorid in Gegenwart von Triethylamin in N,N-Dimethylformamid bei Temperaturen zwischen 0 °C und Raumtemperatur für beispielsweise 18 h.

Die nach dem erfindungsgemäßen Verfahren zugänglichen 1,3-Dioxane weisen vorteilhafte Eigenschaften - zum Beispiel eine große dielektrische Anisotropie $\Delta\varepsilon$ bei vergleichsweise geringer Rotationsviskosität $\gamma_1$ - auf und finden daher breite Verwendung in flüssigkristallinen Medien insbesondere für elektrooptische Anwendungen.

Die nachfolgenden Beispiele veranschaulichen weiter die vorliegende Erfindung, ohne sie in ihrem Umfang zu beschränken.

Vor- und nachstehend bedeuten Prozentangaben Gewichtsprozent. Alle Temperaturen sind in Grad Celsius angegeben. Fp. bedeutet Schmelzpunkt, Klp. Klärpunkt. Ferner bedeuten K beziehungsweise C = kristalliner Zustand, N = nematische Phase, S = smektische Phase und I = isotrope Phase. $S_c$ bedeutet eine smektisch C Phase. $\Delta n$ bezeichnet die optische Anisotropie ($\Delta n = n_e - n_o$, wobei $n_e$ der Brechungsindex des außerordentlichen und $n_o$ der Brechungsindex des ordentlichen Strahls ist) (589 nm, 20 °C). $\Delta\varepsilon$ bezeichnet die dielektrische Anisotropie ($\Delta\varepsilon = \varepsilon_\parallel - \varepsilon_\perp$, wobei $\varepsilon_\parallel$ die Dielektrizitätskonstante parallel zu den Moleküllängsachsen und $\varepsilon_\perp$ die Dielektrizitätskonstante senkrecht dazu bedeutet) (1 kHz, 20 °C). Die optischen Daten wurden bei 20 °C gemessen, sofern nicht ausdrücklich etwas anderes angegeben wird. Die Rotationsviskosität $\gamma_1$ [mPa·s] wurde ebenfalls bei 20 °C bestimmt. SR bezeichnet den spezifischen Widerstand [Ω·cm], VHR die Voltage Holding Ratio. Zur experimentellen Bestimmung der physikalischen Parameter wurde gemäß "Licristal, Physical Properties Of Liquid Crystals, Description of the measurement methods", Hrsg. W. Becker, Merck KGaA, Darmstadt, überarbeitete Ausgabe, 1998, verfahren, wobei die Eigenschaften von Einzelverbindungen zum Teil nach Messung einer definierten Menge der Verbindung (zumeist 10 Gew.-%) in einer definierten Host-Mischung mit bekannten Eigenschaften und anschließende Extrapolation ermittelt wurden.

**Beispiele**

**Beispiel 1**

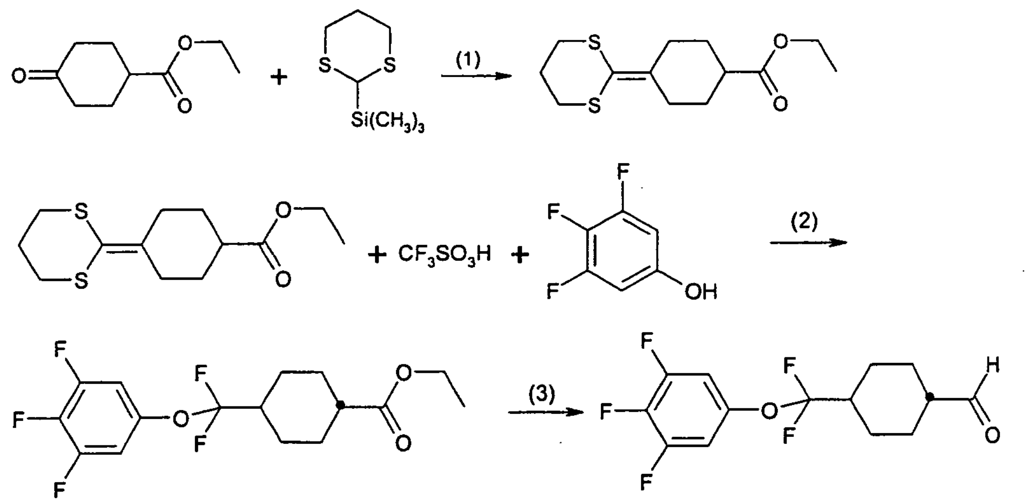


**Stufe (1)**: 230,90 g 2-Trimethylsilyl-1,3-dithian (1,20 mol) werden in THF gelöst, auf -70 °C gekühlt und tropfenweise mit 769,33 ml (1,26 mol) n-Butyllithium (15 %ige Lösung in n-Hexan) versetzt. Man läßt innerhalb von 3 h auf 0 °C erwärmen, kühlt erneut auf -70 °C und tropft diese Lösung vorsichtig zu einer auf -70 °C gekühlten Lösung von 204,25 g (1,20 mol) 4-Cyclohexanoncarbonsäureethylester in 900 ml THF. Nach beendeter Zugabe wird die Kühlung entfernt, und man läßt die Mischung auf Raumtemperatur erwärmen. Die Reaktionsmischung wird auf Eiswasser gegossen und mit Heptan extrahiert. Die vereinigten organischen Phasen werden mit ges. NaCl-Lösung gewaschen und konzentriert, und das so erhaltene gelbe Öl wird aus Pentan umkristallisiert. Hellgelbe Kristalle des Ketendithioketals (78 % Ausbeute), die ohne weitere Reinigung in Stufe (2) eingesetzt werden.

**Stufe (2)**: Das Ketendithioketal aus Stufe (1 ) (128,10 g, 0,47 mol) wird in Dichlormethan gelöst und unter Eiskühlung tropfenweise mit 41,30 ml (0,47 mol) Trifluormethansulfonsäure versetzt. Nach 15 min wird die Kühlung entfernt und 1 h bei Raumtemperatur gerührt. Anschließend wird auf -70 °C gekühlt, eine Mischung von Triethylamin (117,03 ml, 0,84 mol) und 3,4,5-Trifluorphenol (104,53 g, 0,71 mol) in Dichlormethan hinzugegeben und 1 h bei -70 °C gerührt. Dann wird mit 379,22 ml (2,35 mol) Triethylaminhydrofluorid versetzt und nach 5 min Brom (120,48 ml, 2,35 mol) in Dichlormethan innerhalb von 20 min hinzugegeben. Man läßt noch 1 h rühren und den Ansatz auf -10 °C kommen. Dann wird die Lösung unter Rühren auf eine Mischung von 400 ml ges. NatriumhydrogensulfitLösung, 5 l Maschineneis und 800 ml 32 %ige Natronlauge gegeben. Die organische Phase wird abgetrennt und die wäßrige Phase einmal mit Dichlormethan nachextrahiert. Der vereinigte organische Extrakt wird mit Salzlösung gewaschen und zu einem Rückstand eingeengt, der mit Heptan extrahiert und einrotiert wird.

Der so erhaltene Rückstand wird in Toluol/Heptan = 1:1 aufgenommen und über Kieselgel filtriert. Das Eluat wird einrotiert und der Rückstand im Vakuum fraktioniert destilliert. Die Fraktion des gewünschten Produkts (cis/trans-Gemisch) wird aus Pentan in der Kälte umkristallisiert. Auf diese Weise werden 41,6 g (24,5%) des trans-Produkts in einer Reinheit erhalten, die für die weitere Umsetzung ausreichend ist.

Für analytische Zwecke werden 5 g des Rohprodukts erneut in Toluol/Heptan = 1:1 aufgenommen und über basisches Aluminiumoxid filtriert. Die Reinheit des nach Einrotieren und Umkristallisieren erhaltenen Feststoffs beträgt gemäß GC 99,3% des trans-Produkts (Fp.: 39 °C).

**Stufe (3):** Der in Stufe (1) erhaltene Ester (77,50 g, 0,220 mol) wird in Toluol gelöst und auf -70 °C gekühlt. Bei dieser Temperatur werden 261,00 ml (0,261 mol) Diisobutylaluminiumhydrid (DIBAH, 1 M Lösung in Toluol, 0,261 mol) zugetropft. Die Reaktionslösung wird 1 h nachgerührt und dann kalt in gesättigte Ammoniumchlorid-Lösung gegossen. Die organische Phase wird abgetrennt und nochmals mit verdünnter Salzsäure gewaschen, getrocknet, filtriert und eingeengt. Quantitative Ausbeute.

**Beispiel 2**

**Beispiel 2a**

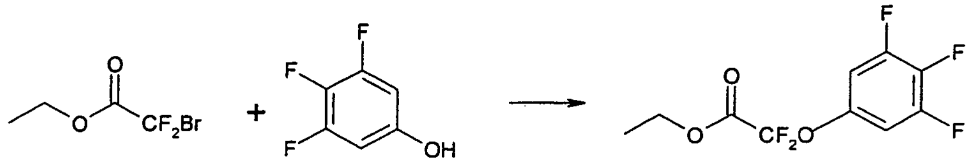

Natriumhydrid (60 %ige Suspension in Mineralöl, 100,00 g, 2,50 mol) wird unter Stickstoffatmosphäre in Tetrahydrofuran (THF) vorgelegt. Hierzu wird eine Lösung von 3,4,5-Trifluorphenol (386,00 g, 2,50 mol) in THF unter schwacher Eiswasserkühlung innerhalb von 2 Stunden zugetropft. Die entstehende Suspension wird für 1 Stunde nachgerührt. Anschließend wird Hexamethylphosphortriamid (22,50 ml, 0,13 mol) zugetropft. Es wird 10 Minuten bei Raumtemperatur nachgerührt, was Lösung 1 ergab.

Ethylbromidfluoracetat (366,00 ml, 2,50 mol) wird in THF gelöst und zu Lösung 1 zugetropft. Das Reaktionsgemisch wird auf 60 °C erwärmt und 16 h bei dieser Temperatur gerührt. Das Gemisch wird eingeengt, der Rückstand in Toluol/Pentan = 1:1 aufgenommen und über Kieselgel filtriert. Nach Vakuumsdestillation und Umkristallisieren wird der gewünschte Ester in einer Ausbeute von 50 % erhalten und ohne weitere Reinigung eingesetzt.

**Beispiel 2b**

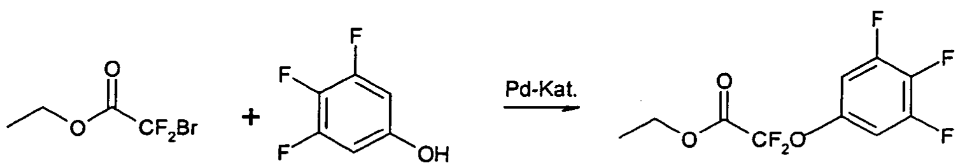


Natriumhydrid (60 %ige Suspension in Mineralöl, 10,00 g, 0,25 mol) wird in Diethylenglycoldimethylether vorgelegt. 3,4,5-Trifluorphenol (38,60 g, 0,25 mol), gelöst in Diethylenglycoldimethylether, wird zu der Natriumhydrid-Suspension zugetropft. Anschließend wird 1 Stunde bei Raumtemperatur nachgerührt (Lösung 1). Während dieser Zeit wird der Katalysator (Bis(tricyclohexylphosphan)-palladiumdichlorid (3,70 g, 0,005 mol) in Diethylenglycoldimethylether vorgelegt und unter Stickstoffatmosphäre mit 10 ml DIBAH (1M in Dichlormethan) svorreduziert. Die Katalysatorlösung wird zu Lösung 1 hinzugegeben, und Ethylbromdifluoracetat (36,60 ml, 0,25 mol) wird innerhalb von 10 Minuten zugetropft. Die Reaktionslösung wird auf 80 °C erwärmt und zwei Stunden bei dieser Temperatur gehalten, dann auf Raumtemperatur gekühlt und in Eiswasser gegossen. Es wird dreimal mit Methyl-t-butylether (MTBE) extrahiert. Die vereinigten organischen Extrakte werden mit Wasser gewaschen, getrocknet und filtriert. Der Rückstand wird eingeengt und dann im Vakuum fraktioniert destilliert. Ausbeute des gewünschten Produkts: 48%. Das erhaltene Produkt wird ohne weitere Reinigung eingesetzt.

**Beispiel 3**

**Beispiel 3a**

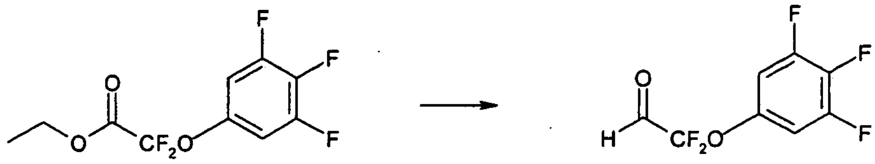

Der in Beispiel 2a beziehungsweise Beispiel 2b hergestellte Ester (0,10 mol) wird in n-Hexan gelöst und auf -70°C abgekühlt. Bei dieser Temperatur wird DIBAH in Hexan (0,10 mol) zugetropft und 4 h nachgerührt. Man läßt auf Raumtemperatur kommen und rührt weitere 96 h nach. Die kalte Reaktionslösung wird in kalte 18 %ige Salzsäure gegossen. Die organische Phase wird abgetrennt und nochmals mit Wasser gewaschen, getrocknet, filtriert und fraktioniert destilliert. Der erfindungsgemäße Aldehyd siedet unterhalb der Siedetemperatur des Ausgangs-Esters und wird in für die weiteren Umsetzungen ausreichender Reinheit erhalten.

**Beispiel 3b**

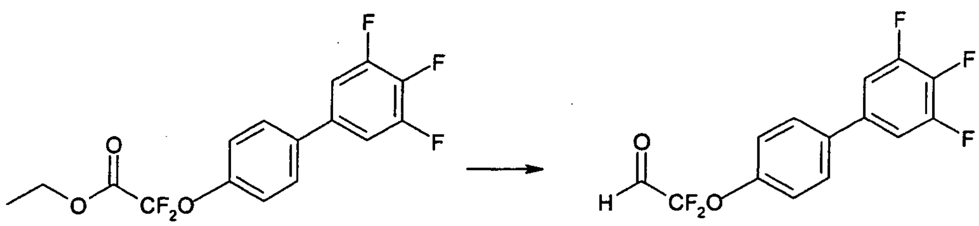

Die Herstellung des Aldehyds erfolgt analog zu Beispiel 3a. Der Aldehyd wird in für die weiteren Umsetzungen ausreichender Reinheit erhalten.

**Beispiel 4**

**Beispiel 4a**

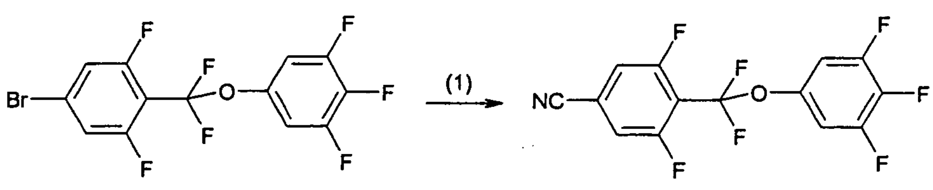

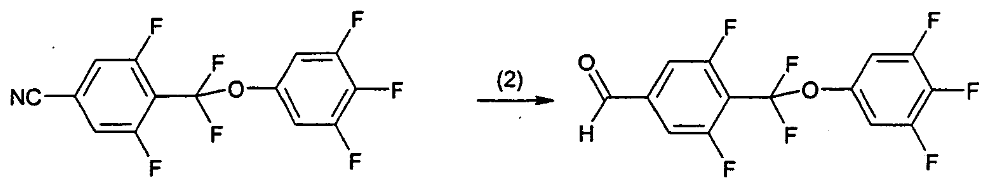


**Stufe (1):** 1-Methyl-2-pyrrolidon (2,4 kg) wird vorgelegt. Zur Trocknung werden ca. 500 ml im Vakuum abdestilliert. Dann werden das Bromid (0,512 mol) und Kupfer(I)-cyanid (0,666 mol) hinzugeben. Man erwärmt bis zur vollständigen Umsetzung auf 150 °C. Nach Abdestillieren des 1-Methyl-2-pyrrolidons wird mit Methyl-tert.-butylether versetzt und mit Wasser extrahiert. Die organische Phase wird eingeengt, der Rückstand in Toluol/Heptan aufgenommen und über Kieselgel filtriert. Das erhaltene Rohprodukt wird aus Heptan umkristallisiert.

**Stufe (2):**

Variante A: Das Produkt aus Stufe (1) (0,015 mol) wird in Toluol vorgelegt und auf -70 °C abgekühlt. Dazu tropft man langsam eine Lösung von Diisobutylaluminiumhydrid in Toluol (0,015 mol), so dass die Temperatur nicht über -70 °C ansteigt. Nach vollständiger Umsetzung wird auf kalte Salzsäure (18 %) gegossen, mit wenig Methyl-tert.-butylether versetzt, die organische Phase abgetrennt, über Natriumsulfat getrocknet, filtriert und eingeengt. Der so erhaltene Aldehyd wird ohne weitere Reinigung eingesetzt.

Variante B: Das Produkt aus Stufe (1) (0,015 mol) wird in Tetrahydrofuran vorgelegt und auf 0 °C abgekühlt. Dazu tropft man langsam eine Lösung von Bis(cyclopentadienyl)zirconiumchloridhydrid in Tetrahydrofuran (0,015 mol), so dass die Temperatur nicht über 0 °C ansteigt. Man rührt 1 h bei 0 °C und dann bei Raumtemperatur bis zur vollständigen Umsetzung nach, gießt auf kalte Salzsäure, versetzt mit wenig Methyl-tert.-butylether, trennt die organische Phase ab, trocknet, filtriert und engt ein. Der so erhaltene Aldehyd wird ohne weitere Reinigung eingesetzt.

**Beispiel 4b**

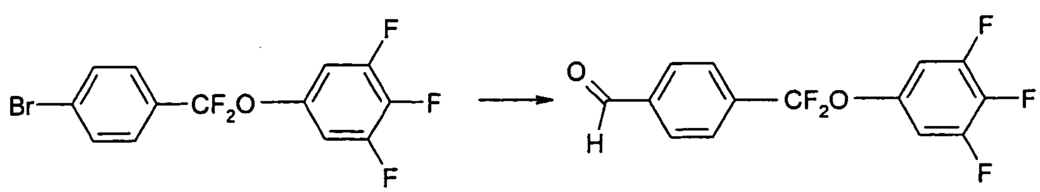

Unter Stickstoffatmosphäre wurde in 10 l THF unter Rühren 2 M Isopropylmagnesiumchlorid-Lösung in THF (900ml) vorgelegt. Dann tropfte man eine Lösung des Bromaromaten (585 g) in THF vorsichtig zu und rührte 1 h bei 20 °C nach. Anschließend wurde eine Lösung von n-Formylpiperidin (206 g) in THF über 20 min zugetropft. Der Ansatz wurde noch 1 h bei 20 °C nachgerührt und dann mit 1 M HCl (4 l) versetzt. Die Phasen wurden getrennt und die wässrige Phase mit Toluol extrahiert. Die vereinigten organischen Phasen wurden mit Wasser gewaschen, getrocknet, filtriert und im Vakuum eingeengt.

Der ölige Rückstand wurde mit Petrolether gerührt, wobei hellgelbe Kristalle ausfielen, die abgesaugt, gewaschen und getrocknet wurden. Aus der Mutterlauge kristallisierte weiteres Produkt aus, das ebenfalls abgesaugt, gewaschen und getrocknet wurde. Hellgelbe Kristalle, Fp. 47-50 °C. Ausbeute (nicht optimiert): 337 g (77%).

**Beispiel 5**

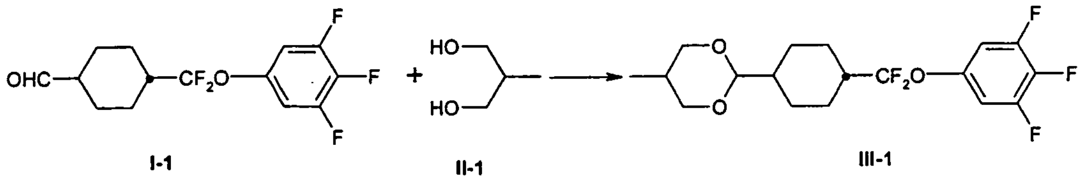

**I-1** **II-1** **III-1**

2,80 g (31,10 mmol) 2-Methyl-1,3-propandiol (**II-1**), 9,60 g (31,14 mmol) des Aldehyds **I-1** aus Beispiel 1 und 0,63 g (3,30 mmol) p-Toluolsulfonsäure werden in Toluol gelöst und für 30 min am Wasserabscheider zum Rückfluß erhitzt. Nach Abkühlen auf Raumtemperatur wird mit Wasser, dann mit ges. $NaHCO_3$-Lösung gewaschen. Die organische Phase wird eingeengt. Der verbleibende Rückstand wird in Toluol/Heptan = 1:1 aufgenommen und über Kieselgel filtriert, und das Eluat wird erneut im Vakuum eingeengt. Nach Umkristallisieren aus Heptan wird der Feststoff in Toluol/Heptan gelöst und über Kieselgel und basisches Aluminiumoxid filtriert. Nach Einengen im Vakuum und erneuter Umkristallisation aus Heptan wird trans-**III-1** erhalten (3,3 g), das gemäß HPLC-Analyse rein ist.

**Beispiele 6-10**

In zu Beispiel 5 analoger Weise werden die Dioxane **III-2** bis **III-6** hergestellt:

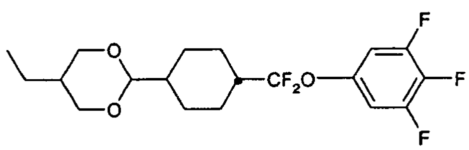

**III-2**

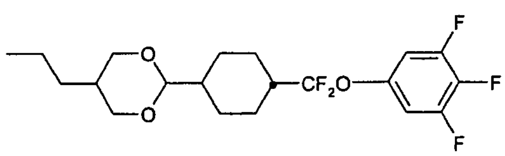

**III-3**

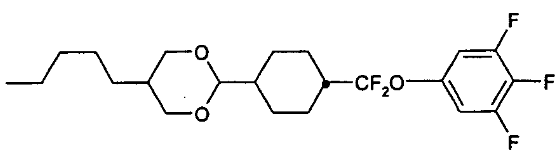

**III-4**

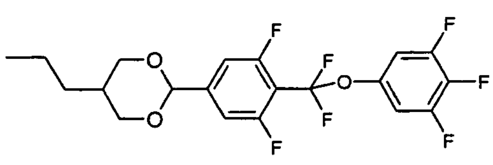

**III-5**

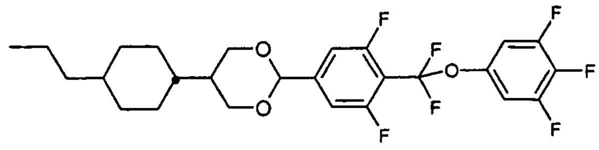

III-6

Wichtige physikalische Eigenschaften der Dioxane III-1 bis III-4 sind in der folgenden Tabelle wiedergegeben.

Tabelle

| **Dioxan** | $\Delta n$ | $\Delta\varepsilon$ | **Klp. [°C]** | $\gamma_1$ **[mPa·s]** |
|---|---|---|---|---|
| **III-1** | 0,0689 | 22,7 | 7,9 | nicht bestimmt |
| **III-2** | 0,0618 | 21,3 | 27,9 | nicht bestimmt |
| **III-3** | 0,0688 | 20,6 | 45,3 | 207 |
| **III-4** | 0,0642 | 20,1 | 54,8 | 229 |

Beispiel 11

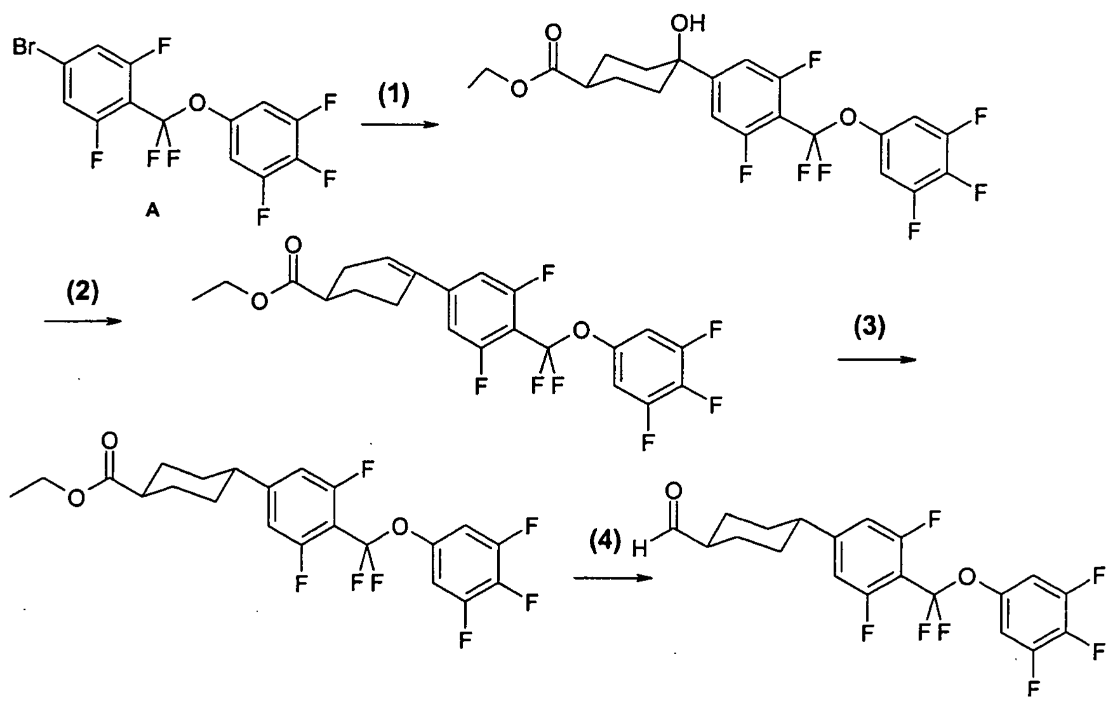


Stufe 1:

Eine Lösung von 58 g des Bromaromaten **A** in THF wird zu einer Lösung von 1,2 Äquivalenten Isopropylmagnesiumchlorid in THF bei ~ 20 °C zugetropft. Die Reaktionslösung wird ca. 40 Minuten bei Raumtemperatur nachgerührt. Dann wird zu einer Lösung von 1 Äquivalent Cyclohexan-4-oncarbonsäure-ethylester in THF bei Raumtemperatur unter Wasserkühlung zugetropft. Die Umsetzung wird nachgerührt. Anschließend wird mit verdünnter HCl-Lösung zersetzt, die Phasen getrennt, die organische Phase mit Wasser gewaschen und im Vakuum zum Rückstand

aufkonzentriert. Das Rohprodukt (76 g) kann ohne weitere Reinigung in der nächsten Stufe eingesetzt werden.

Stufe 2:

Eine Lösung von 26 g des Produkts aus Stufe 1 in Toluol wird auf ~ 70 °C erwärmt und mit 1 g Schwefelsäure 95% - 98%-ig versetzt. Die Reaktionslösung wird ca. 3 Stunden bei ~ 80 °C eingerührt. Die Lösung wird abgekühlt und bei ~ 45 °C mit Wasser versetzt. Die wässrige Phase wird abgetrennt, die organische Phase mit Wasser gewaschen und im Vakuum zum Rückstand aufkonzentriert. Zur Reinigung wird das Rohprodukt mit Toluol über Kieselgel chromatographiert. Die produkthaltigen Fraktionen werden zum Rückstand einrotiert. Das Produkt (14 g) kann ohne weitere Reinigung in der nächsten Stufe eingesetzt werden.

Stufe 3:

Eine Lösung von 10 g des Produkts aus Stufe 2 in THF wird mit 5% Pt/C drucklos ca. 17 Stunden hydriert. Die Hydrierlösung wird im Vakuum zum Rückstand aufkonzentriert und das Rohprodukt über Kieselgel mit Toluol filtriert. Die produkthaltigen Fraktionen werden erneut im Vakuum zum Rückstand einrotiert. Das Produkt (9,8 g) kann ohne weitere Reinigung in der nächsten Stufe eingesetzt werden.

Stufe 4:

Eine Lösung von 6,5 g des Produkts aus Stufe 3 in Toluol/THF 4:1 (Gew./Gew.) wird auf ca - 76 °C gekühlt und 1,2 Äquivalente einer 20%igen Lösung von Diisobutylaluminiumhydrid in n-Hexan langsam bei dieser Temperatur zugetropft. Die Reaktionslösung wird nach ca. 4,5 h in eine kalte wässrige Ammoniumchloridlösung eingetropft und das Gemisch ca. 5 Minuten gerührt. Dann werden die Phasen getrennt, die wässrige Phase mit Toluol nachextrahiert und die vereinten organischen Phasen zuerst mit 1 N HCl-Lösung und dann mit Wasser gewaschen. Die organische Phase wird im Vakuum zum Rückstand aufkonzentriert. Das Rohprodukt wird durch Chromatographie an Kieselgel mit Toluol/EtOAc 9:1 aufgereinigt. Die produkthaltigen Fraktionen werden im Vakuum zum Rückstand einrotiert. Das cis/trans-Isomerengemisch wird in Methanol mit 0,1 Äquivalenten NaOH als Base isomerisiert. Das Gemisch wird mit Toluol und Wasser versetzt, die Phasen getrennt, die organische Phase mit Wasser gewaschen und im Vakuum zum Rückstand aufkonzentriert. Durch Kristallisation aus Isopropanol, chromatographische Aufreinigung der Mutterlaugenrückstände und erneute Kristallisation aus Isopropanol wird die reine trans-Verbindung erhalten. Ausbeute: 3,8 g.

## Patentansprüche

**1.** Aldehyd gemäß der allgemeinen Formel I

$$OHC\text{-}(A^{11})_m\text{-}Z^{11}\text{-}(A^{12}\text{-}Z^{12})_n\text{-}(A^{13}\text{-}Z^{13})_p\text{-}A^{14}$$

wobei

$A^{11}$, $A^{12}$ und $A^{13}$ unabhängig voneinander für

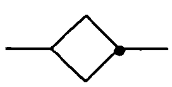

,

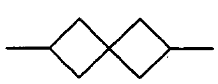

,

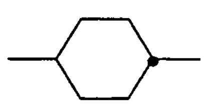

oder

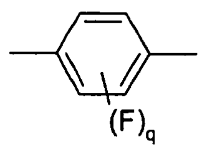

| | |
|---|---|
| | stehen; |
| $A^{14}$ | für |

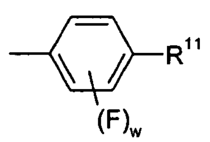

| | |
|---|---|
| | steht; |
| $Z^{11}$, $Z^{12}$ und $Z^{13}$ | unabhängig voneinander eine Einfachbindung, $-CH_2CH_2-$, $-CF_2CH_2-$, $-CH_2CF_2-$, $-CF_2CF_2-$, $-CH=CH-$, $-C\equiv C-$, -CO-O-, -O-CO-, $-CH_2O-$, $-OCH_2-$ oder $-CF_2O-$ bedeuten, wobei wenigstens eines von $Z^{11}$, $Z^{12}$ und $Z^{13}$ $-CF_2O-$ bedeutet; |
| m, n und p | unabhängig voneinander 0 oder 1 bedeuten; |
| q und w | unabhängig voneinander 0, 1, 2, 3 oder 4 bedeuten; |
| $R^{11}$ | H, F, Cl, Br, I, CN, -NCS, einen O-Aralkylrest oder einen unsubstituierten oder mit F, Cl, Br, I und/oder - CN ein- oder mehrfach substituierten Alkylrest mit 1 bis 15 Kohlenstoffatomen bedeutet, wobei in diesem Rest eine oder mehrere $CH_2$-Gruppen jeweils unabhängig voneinander durch $-C\equiv C-$, - CH=CH-, -O-, -S-, -CO-, -CO-O- oder -O-CO- so ersetzt sein können, dass Heteroatome nicht direkt miteinander verknüpft sind. |

2. Aldehyd gemäß Anspruch 1, **dadurch gekennzeichnet, dass** $A^{14}$ für

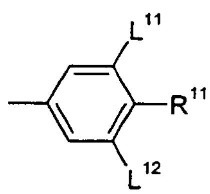

steht, wobei $R^{11}$ wie in Anspruch 1 definiert ist und $L^{11}$ und $L^{12}$ unabhängig voneinander H oder F bedeuten.

3. Aldehyd gemäß Anspruch 2, **dadurch gekennzeichnet, dass** $R^{11}$ H, F, Cl, $OCF_3$, $OCHF_2$ oder $CF_3$ bedeutet.

4. Aldehyd gemäß einem der vorangehenden Ansprüche, **dadurch gekennzeichnet, dass** $Z^{11}$, $Z^{12}$ und $Z^{13}$ unabhängig voneinander eine Einfachbindung, $-CH_2CH_2-$, $-CF_2CH_2-$, $-CH_2CF_2-$, $-CF_2CF_2-$ oder $-CF_2O-$ insbesondere eine Einfachbindung oder $-CF_2O-$ bedeuten, wobei wenigstens eines von $Z^{11}$, $Z^{12}$ und $Z^{13}$ $-CF_2O-$ bedeutet.

5. Aldehyd gemäß einem der vorangehenden Ansprüche, **dadurch gekennzeichnet, dass** m 0 ist.

6. Aldehyd gemäß einem der vorangehenden Ansprüche, **dadurch gekennzeichnet, dass** $Z^{11}$ $-CF_2O-$ bedeutet.

7. Aldehyd gemäß einem der vorangehenden Ansprüche, **dadurch gekennzeichnet, dass** n und p 0 sind.

8. Aldehyd gemäß Anspruch 1, **dadurch gekennzeichnet, dass** der Aldehyd ausgewählt ist aus der Gruppe beste-

hend aus:

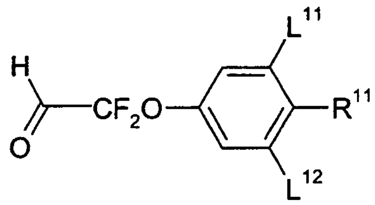

I-A

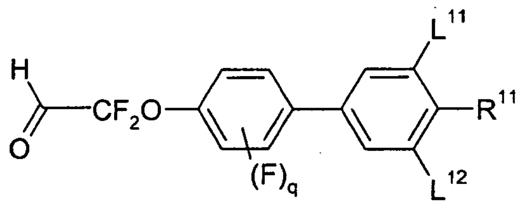

I-B

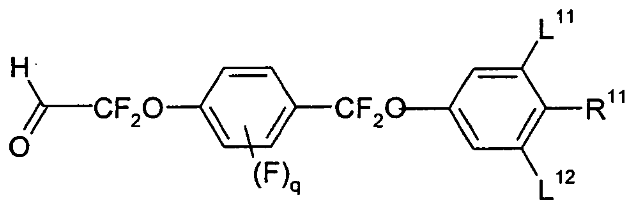

I-C

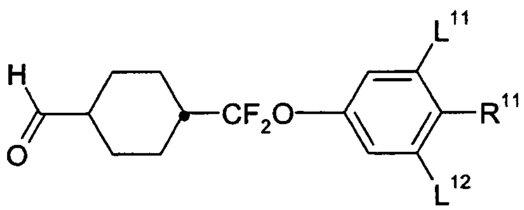

I-D

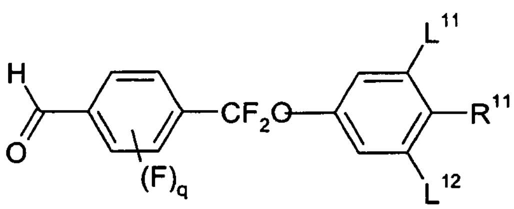

I-E

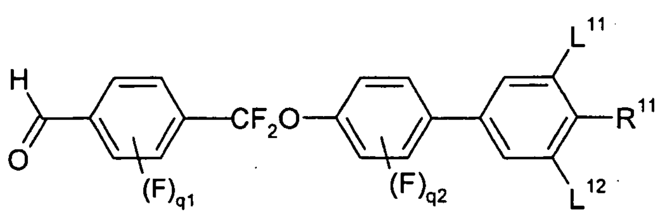

I-F

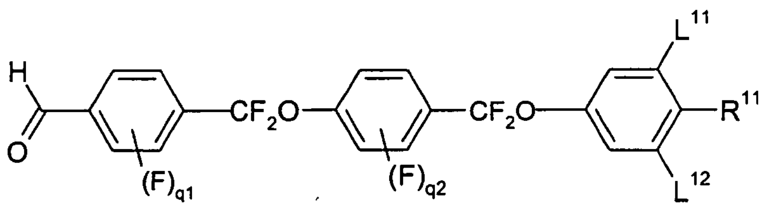

I-G

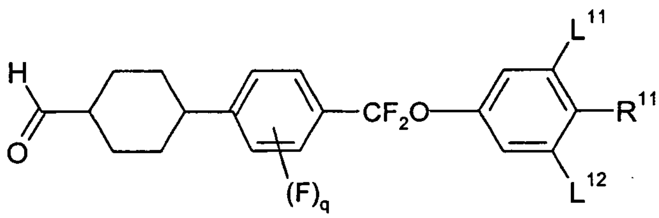

I-H

wobei $R^{11}$, $L^{11}$, $L^{12}$ und q wie in einem der vorangehenden Ansprüche definiert sind und q1 und q2 unabhängig voneinander 0, 1, 2, 3 oder 4 bedeuten.

**9.** Verfahren zur Herstellung einer 1,3-Dioxanverbindung, **dadurch gekennzeichnet, dass** ein Aldehyd gemäß einem der vorangehenden Ansprüche mit einem 1,3-Diol oder einem 1,3-bis-silylierten Derivat des 1,3-Diols umgesetzt wird.

**10.** Verfahren gemäß Anspruch 9, **dadurch gekennzeichnet, dass** das 1,3-Diol oder das 1,3-bis-silylierte Derivat des 1,3-Diols eine Verbindung der Formel II ist:

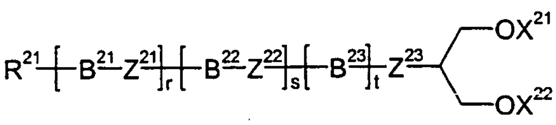

II

wobei

| | |
|---|---|
| r, s und t | unabhängig voneinander 0 oder 1 bedeuten; |
| $R^{21}$ | einen unsubstituierten oder mit F, Cl, Br, I und/oder - CN ein- oder mehrfach substituierten Alkylrest mit 1 bis 15 Kohlenstoffatomen bedeutet, wobei in diesem Rest eine oder mehrere $CH_2$-Gruppen jeweils unabhängig voneinander durch -C≡C-, - CH=CH-, -O-, -S-, -CO-, -CO-O- oder -O-CO- so ersetzt sein können, dass Heteroatome nicht direkt miteinander verknüpft sind; |
| $B^{21}$, $B^{22}$ und $B^{23}$ | unabhängig voneinander |

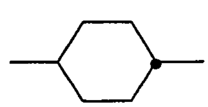

oder

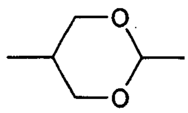

| | |
|---|---|
| | bedeuten; |
| $Z^{21}$, $Z^{22}$ und $Z^{23}$ | unabhängig voneinander eine Einfachbindung, $-CH_2CH_2-$, $-CF_2CH_2-$, $-CH_2CF_2-$, $-CF_2CF_2-$, - CH=CH- oder -C≡C- bedeuten; |
| $X^{21}$ und $X^{22}$ | H oder $SiR^{22}R^{23}R^{24}$ bedeuten, |
| wobei $R^{22}$, $R^{23}$ und $R^{24}$ | jeweils unabhängig voneinander ein Alkanylrest mit 1 bis 6 Kohlenstoffatomen ist und einer oder zwei der Reste $R^{22}$, $R^{23}$ und $R^{24}$ auch Phenyl sein können. |

**11.** Verfahren gemäß einem der Ansprüche 9 oder 10, **dadurch gekennzeichnet, dass** der Aldehyd eine Struktur der Formel Ia aufweist:

$$OHC\text{-}(A^{11})_m\text{-}CF_2O\text{-}(A^{12}\text{-}Z^{12})_n\text{-}(A^{13}\text{-}Z^{13})_p\text{-}A^{14} \qquad \text{Ia}$$

wobei m 0 ist und $A^{12}$, $A^{13}$, $A^{14}$, $Z^{12}$, $Z^{13}$, n und p die in einem der Ansprüche 1 bis 7 angegebene Bedeutung aufweisen.

**12.** Verfahren gemäß einem der Ansprüche 9 oder 10, **dadurch gekennzeichnet, dass** der Aldehyd eine Struktur der Formel Ia aufweist:

$$OHC\text{-}(A^{11})_m\text{-}CF_2O\text{-}(A^{12}\text{-}Z^{12})_n\text{-}(A^{13}\text{-}Z^{13})_p\text{-}A^{14} \qquad \text{Ia}$$

wobei m 1 ist, $A^{11}$ einen 1,4-Cyclohexylenring darstellt und $A^{12}$, $A^{13}$, $A^{14}$, $Z^{12}$, $Z^{13}$, n und p die in einem der Ansprüche 1 bis 7 angegebene Bedeutung aufweisen.

Europäisches Patentamt

# EUROPÄISCHER RECHERCHENBERICHT

Nummer der Anmeldung
EP 04 01 0356

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | ISEKI K ET AL: "Catalytic Asymmetric Nitroaldol Reaction of alpha,alpha-Difluoro Aldehydes Mediated by Rare Earth-Lithium-BINOL Complexes" TETRAHEDRON LETTERS, ELSEVIER SCIENCE PUBLISHERS, AMSTERDAM, NL, Bd. 37, Nr. 50, 9. Dezember 1996 (1996-12-09), Seiten 9081-9084, XP004070564 ISSN: 0040-4039 * Seite 9083; Beispiel 1e; Tabelle 2 * | 1,2,4-8 | C09K19/34 C07D319/06 C07C47/47 C07C47/277 C07C47/575 |
| X | DE 102 43 776 A (MERCK PATENT GMBH) 10. April 2003 (2003-04-10) RN 509089-20-7 * Seite 53; Beispiel G * Schema 1-4 * Seiten 20-23 * | 1-12 | |
| X | DE 102 29 476 A (MERCK PATENT GMBH) 13. Februar 2003 (2003-02-13) | 1,2,4-9 | |
| Y | Schema 3 * Seite 21 * | 9-12 | **RECHERCHIERTE SACHGEBIETE (Int.Cl.7)** |
| Y | DE 102 17 771 A (MERCK PATENT GMBH) 31. Oktober 2002 (2002-10-31) * Anspruch 7 * | 9-12 | C09K C07D C07C |
| Y | US 5 997 766 A (KIRSCH PEER ET AL) 7. Dezember 1999 (1999-12-07) Step 1.6 * Spalten 11-12 * Reaction Scheme * Spalten 5-6 * | 9-12 | |
| E | EP 1 416 030 A (MERCK PATENT GMBH) 6. Mai 2004 (2004-05-06) RN 685563-70-6 * Seite 61, letzter Absatz; Beispiel 7 * | 1-9 | |

Der vorliegende Recherchenbericht wurde für alle Patentansprüche erstellt

| Recherchenort | Abschlußdatum der Recherche | Prüfer |
|---|---|---|
| München | 14. Juli 2004 | Kiernan, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 04 01 0356

*In diesem Anhang sind die Mitglieder der Patentfamilien der im obengenannten europäischen Recherchenbericht angeführten Patentdokumente angegeben.*
*Die Angaben über die Familienmitglieder entsprechen dem Stand der Datei des Europäischen Patentamts am*
*Diese Angaben dienen nur zur Unterrichtung und erfolgen ohne Gewähr.*

14-07-2004

| Im Recherchenbericht angeführtes Patentdokument | Datum der Veröffentlichung | Mitglied(er) der Patentfamilie | Datum der Veröffentlichung |
|---|---|---|---|
| DE 10243776 A | 10-04-2003 | DE 10243776 A1<br>JP 2003176251 A<br>US 2003213935 A1 | 10-04-2003<br>24-06-2003<br>20-11-2003 |
| DE 10229476 A | 13-02-2003 | DE 10229476 A1<br>GB 2379442 A ,B<br>JP 2003176265 A<br>US 2003216554 A1 | 13-02-2003<br>12-03-2003<br>24-06-2003<br>20-11-2003 |
| DE 10217771 A | 31-10-2002 | DE 10217771 A1 | 31-10-2002 |
| US 5997766 A | 07-12-1999 | DE 19654487 A1 | 02-07-1998 |
| EP 1416030 A | 06-05-2004 | DE 10348172 A1<br>EP 1416030 A1 | 13-05-2004<br>06-05-2004 |

EPO FORM P0461

Für nähere Einzelheiten zu diesem Anhang : siehe Amtsblatt des Europäischen Patentamts, Nr.12/82